Europäisches Patentamt
European Patent Office
Office européen des brevets

(19) (11) Publication number: **0 299 620 A1**

(12) **EUROPEAN PATENT APPLICATION**

(21) Application number: 88305285.4

(22) Date of filing: 09.06.88

(51) Int. Cl.⁴: **C07D 417/06 , C07D 417/14 , A61K 31/425**

Claim for the following Contracting State: ES.

The title of the invention has been amended (Guidelines for Examination in the EPO, A-III, 7.3).

(30) Priority: **13.06.87 GB 8713861**

(43) Date of publication of application: **18.01.89 Bulletin 89/03**

(84) Designated Contracting States: **AT BE CH DE ES FR GB GR IT LI LU NL SE**

(71) Applicant: **BEECHAM GROUP PLC**
**Beecham House Great West Road**
**Brentford Middlesex TW8 9BD(GB)**

(72) Inventor: **Haigh, David Beecham Pharmaceuticals**
**Great Burgh Yew Tree Bottom Road**
**Epsom Surrey, KT18 5XQ(GB)**
Inventor: **Hindley, Richard Mark Beecham Pharmaceuticals**
**Great Burgh Yew Tree Bottom Road**
**Epsom Surrey, KT18 5XQ(GB)**

(74) Representative: **Rutter, Keith et al**
**Beecham Pharmaceuticals Great Burgh Yew Tree Bottom Road**
**Epsom Surrey KT18 5XQ(GB)**

(54) **Thiazolidinedione derivatives.**

(57) A compound of formula (I):

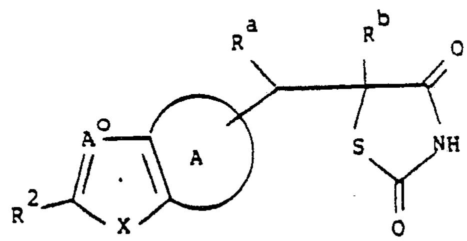

(I)

or a pharmaceutically acceptable salt thereof, wherein: $A^0$ represents nitrogen or a moiety $R^1$- C -
||

wherein
$R^1$ represents hydrogen, alkyl or a substituted or unsubstituted aryl group;
$R^2$ represents a moiety $R^3$-Y-Z- wherein $R^3$ represents substituted or unsubstituted phenyl, substituted or unsubstituted pyridyl or a substituted or unsubstituted oxazolyl group, and Y represents $-(CH_2)_n$- wherein n represents zero or any integer in the range of 1 to 6 and Z represents $-CH_2$-,-CH(OH)- or -CO-;
$R^a$ and $R^b$ each represent hydrogen or $R^a$ and $R^b$ together represent a bond;
A represents a residue of a benzene ring, the carbon atoms of the residue having in total up to four substituents;
and X represents O or S; a process for preparing such a compound, a pharmaceutical composition

EP 0 299 620 A1

containing such a compound and the use of the compound and composition in medicine.

## Novel Compounds.

This invention relates to a class of novel thiazolidinedione derivatives, to a process for preparing such compounds, to pharmaceutical compositions containing such compounds and the use of such compounds and compositions in medicine.

European Patent Applications, Publication Numbers 0177353 and 0193256 disclose certain compounds as therapeutic agents against diabetes and hyperlipaemia.

It has surprisingly been discovered that certain novel thiazolidinedione derivatives show good blood-glucose and blood-lipid lowering activity and are therefore of potential use in the treatment and/or prophylaxis of hyperglycaemia and hyperlipidaemia.

Accordingly, the present invention provides a compound of formula (I):

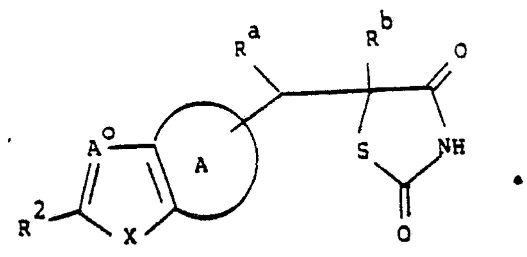

(I)

or a pharmaceutically acceptable salt thereof, or a pharmaceutically acceptable solvate thereof, wherein:
$A^0$ represents nitrogen or a moiety $R^1-\underset{\|}{C}-$

wherein
$R^1$ represents hydrogen, alkyl or a substituted or unsubstituted aryl group;
$R^2$ represents a moiety $R^3$-Y-Z- wherein $R^3$ represents substituted or unsubstituted phenyl, substituted or unsubstituted pyridyl or a substituted or unsubstituted oxazolyl group, and Y represents $-(CH_2)_n-$ wherein n represents zero or any integer in the range of 1 to 6 and Z represents $-CH_2-$,-CH(OH)- or -CO-;
$R^a$ and $R^b$ each represent hydrogen or $R^a$ and $R^b$ together represent a bond;
A represents a residue of a benzene ring, the carbon atoms of the residue having in total up to four substituents;
and X represents O or S.

Suitably, $A^0$ represents nitrogen.
Suitably, $A^0$ represents a moiety $R^1-\underset{\|}{C}-$

Preferably $A^0$ represents a moiety $R^1-\underset{\|}{C}-$

Suitably, $R^1$ represents hydrogen, $C_{1-}$ alkyl or phenyl.
Favourably, $R^1$ represents $C_{1-6}$ alkyl, especially methyl.
Favourably, $R^1$ represents phenyl.
Preferably, $R^1$ represents hydrogen.
Suitably, $R^2$ represents a moiety $R^3$-Z-.
Favourably, $R^2$ represents $R^3-CH_2-$.
Favourably, $R^2$ represents $R^3$-CH(OH)-.
Favourably, $R^2$ represents $R^3$-CO-.
Suitably, $R^3$ represents substituted or unsubstituted phenyl.
Suitably, $R^3$ represents substituted or unsubstituted pyrid-2-yl.
Favourably, $R^3$ represents substituted or unsubstituted oxazolyl.
When $R^3$ represents substituted or unsubstituted oxazolyl, it is suitably substituted or unsubstituted oxazol-4-yl or substituted or unsubstituted oxazol-5-yl.
Preferably, $R^3$ represents a substituted or unsubstituted oxazol-4-yl group.
When $R^3$ represents oxazolyl, it may conveniently be represented by a moiety of formula (a):

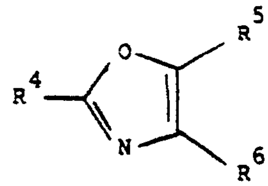

(a)

wherein $R^4$ represents an alkyl group or substituted or unsubstituted aryl and either $R^5$ represents a bond and $R^6$ represents an alkyl group, suitably a $C_{1-6}$ alkyl group, or $R^5$ represents an alkyl group, suitably a $C_{1-6}$ alkyl group, and $R^6$ represents a bond.

Suitably, $R^4$ represents a $C_{1-6}$ alkyl group or a substituted or unsubstituted phenyl group.

Preferably, $R^5$ represents an alkyl group, suitably a $C_{1-6}$ alkyl group, and $R^6$ represents a bond.

Preferably, when either of $R^5$ or $R^6$ represents alkyl, the alkyl group is a methyl group.

Preferably, $R^a$ and $R^b$ each represent hydrogen.

Preferably X represents O.

In a preferred aspect the present invention provides a class of compounds, falling wholly within the scope of formula (I), of formula (II):

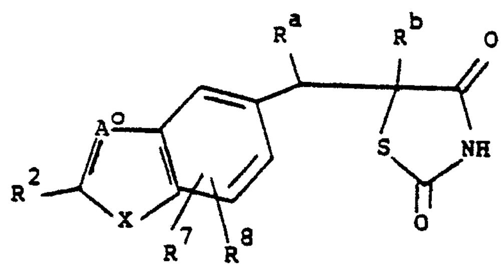

(II)

or a pharmaceutically acceptable salt thereof, or a pharmaceutically acceptable solvate thereof, wherein $A^0$, $R^2$, $R^a$, $R^b$ and X are as defined in relation to formula (I) and $R^7$ and $R^8$ each independently represent hydrogen, alkyl, alkoxy or halogen.

Suitably, $R^7$ represents hydrogen.

Suitably, $R^8$ represents hydrogen, alkyl, alkoxy or halogen.

When $R^7$ or $R^8$ represents other than hydrogen, it is preferred that at least one of $R^7$ and $R^8$ is substituted ortho to the carbon atom attached to X, generally this will be $R^8$.

Favourably, $R^8$ represents hydrogen or alkoxy.

Preferably, $R^8$ represents hydrogen or methoxy.

When used herein the term 'aryl' includes phenyl and naphthyl optionally substituted with up to five, preferably up to three, groups selected from halogen, alkyl, phenyl, alkoxy, haloalkyl, hydroxy, amino, nitro, carboxy, alkoxycarbonyl, alkoxycarbonylalkyl, alkylcarbonyloxy or alkylcarbonyl groups.

Suitable optional substituents for the phenyl group represented by $R^3$ include those referred to above in relation to the term aryl, but in particular halogen and amino should be mentioned.

Suitable optional substituents for the oxazolyl group represented by $R^3$ include those referred to above in relation to the term aryl but in particular alkyl and phenyl should be mentioned.

When used herein the term 'halogen' refers to fluorine, chlorine, bromine and iodine; preferably chlorine.

When used herein the term 'alkyl', or 'alkoxy' relates to groups having straight or branched carbon chains containing up to 12 carbon atoms.

Suitable alkyl groups are $C_{1-12}$ alkyl groups especially $C_{1-6}$ alkyl groups e.g. methyl, ethyl, n-propyl, iso-propyl, n-butyl, isobutyl or tert-butyl groups.

Suitable pharmaceutically acceptable salts include acid addition salts, salts of the thiazolidinedione moiety, especially the nitrogen atom thereof, and where appropriate salts of carboxy groups.

Suitable pharmaceutically acceptable acid addition salts of compound (I) include pharmaceutically acceptable inorganic salts such as the sulphate, nitrate, phosphate, borate, hydrochloride and hydrobromide and pharmaceutically acceptable organic acid addition salts such as acetate, tartrate, maleate, citrate, succinate, benzoate, ascorbate, methane-sulphonate, α-keto glutarate, α-glycerophosphate, and glucose-1-phosphate.

Preferably the acid addition salt is a hemisuccinate, hydrochloride, α-ketoglutarate, α-glycerophosphate or glucose-1-phosphate, in particular the hydrochloride salt.

Suitable pharmaceutically acceptable salts of the thiazolidinedione moiety include metal salts, especially the alkali metal salts such as the lithium, sodium and potassium salts.

Suitable pharmaceutically acceptable salts of carboxy groups include metal salts, such as for example aluminium, alkali metal salts such as sodium or potassium, alkaline earth metal salts such as calcium or magnesium and ammonium or substituted ammonium salts, for example those with lower alkylamines such as triethylamine, hydroxy-lower alkylamines such as 2-hydroxyethylamine, bis-(2-hydroxyethyl)-amine or tri-(2-hydroxyethyl) -amine, cycloalkylamines such as bicyclohexylamine, or with procaine, dibenzylpiperidine, N-benzyl-β-phenethylamine, dehydroabietylamine, N,N′-bisdehydroabietylamine, glucamine, N-methylglucamine or bases of the pyridine type such as pyridine, collidine or quinoline.

Suitable pharmaceutically acceptable solvates include hydrates.

The present invention also provides a process for the preparation of a compound of formula (I), or a pharmaceutically acceptable salt thereof, or a pharmaceutically acceptable solvate thereof, which process comprises reacting a compound of formula (III):

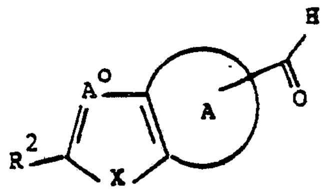

(III)

wherein $A^0$, A, $R^2$ and X are as defined in relation to formula (I), with 2,4-thiazolidinedione; and thereafter, if required, carrying out one or more of the following optional steps:

(i) converting a compound of formula (I) into a further compound of formula (I);

(ii) preparing a pharmaceutically acceptable salt of a compound of formula (I) or a pharmaceutically acceptable solvate thereof.

The reaction between the compound of formula (III) and 2,4-thiazolidinedione may be carried out under any convenient reaction conditions, for example in a solvent, such as toluene, at an elevated temperature, preferably at the reflux temperature of the solvent and preferably in the presence of a suitable catalyst such as piperidinium benzoate. Suitably, in the reaction between compound (III) and 2,4-thiazolidinedione, the water produced in the reaction is removed from the reaction mixture, for example by means of a Dean and Stark apparatus.

A compound of formula (III) may be prepared by reacting a compound of formula (IV):

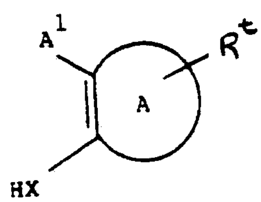

(IV)

wherein A and X are as defined in relation to formula (I), $R^t$ represents a carbonyl group or a protected form thereof and $A^1$ represents $NH_2$ or a moiety

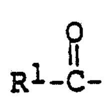

wherein $R^1$ is as defined in relation to formula (I), with a compound of formula (V):

$R^2$-$A^2$ (V)

and either when $A^1$ represents $NH_2$,

$R^2$ is as defined in relation to formula (I) and $A^2$ represents CO-$R^x$ wherein $R^x$ is halogen, hydroxy or alkoxy for the preparation of compounds of formula (III) wherein $A^0$ represents nitrogen, or when $A^1$ represents

$R^1-\overset{O}{\overset{\|}{C}}-$, $R^2$ represents $R^3$-Y-CO- wherein $R^3$ and Y are as defined in relation to formula (I) and $A^2$ represents $-CH_2-R^y$ wherein $R^y$ is a leaving group for the preparation of compounds of formula (III) wherein $A^0$ represents a moiety $R^1-\underset{\|}{C}-$; and thereafter, if required, converting a compound of formula (III) into a further compound of formula (III) and removing any protecting group.

Preferably, in the compound of formula (V), $R^3$ represents $R^{3a}$ wherein $R^{3a}$ is substituted or unsubstituted phenyl or a substituted or unsubstituted oxazolyl group.

Preferably, $R^t$ represents a carbonyl protecting group when $A^1$ represents $NH_2$. When $R^t$ represents a carbonyl protecting group any conventional protecting group may be used, for example an ethylene acetal protecting group. The said protecting group may be prepared and removed using conventional procedures, for example the ethylene acetal may be removed via hydrolysis under mildly acidic conditions.

Conveniently, $R^x$ represents hydroxy. A suitable leaving group $R^y$ is a halogen atom, in particular a bromine atom.

The reaction between the compounds of formula (IV) and (V) may be carried out using any convenient reaction conditions, for example for compounds of formula (III) wherein $A^0$ represents nitrogen the compound of formula (IV) and the appropriate compound of formula (V) may be reacted together in any suitable inert solvent, such as xylene, suitably at an elevated temperature, such as the boiling point of the solvent, and conveniently the water produced in the reaction is removed from the reaction, for example in a Dean and Stark apparatus. Also for compounds of formula (III) wherein $A^0$ represents $R^1-\underset{\|}{C}-$ the compound of formula (IV) and the appropriate compound of formula (V) are reacted together in a solvent such as 2-butanone, at an elevated temperature, preferably the reflux temperature of the solvent, and preferably in the presence of potassium carbonate.

The compounds of formula (IV) and (V) are either known compounds or they may be prepared by methods analogous to those used to prepare known compounds, for example those disclosed in European Patent No. 0177353.

A compound of formula (III) wherein $A^0$ represents $R^1-\underset{\|}{C}-$ may also be prepared by reacting a compound of formula (VI):

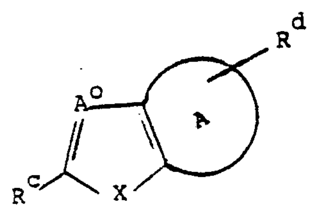

(VI)

wherein $A^0$ is $R^1-\underset{\|}{C}-$ and X is as defined in relation to formula (I), $R^c$ is a moiety convertible to the abovedefined moiety $R^2$ and $R^d$ represents a carbonyl group or a protected form thereof, with an appropriate reagent capable of converting a moiety $R^c$ into the said moiety $R^2$; and thereafter, if required, carrying out one or more of the following steps:

(i) removing any carbonyl protecting group; and

(ii) converting a compound of formula (III) into a further compound of formula (III).

The particular nature of the compound of formula (VI) and the nature of the appropriate reagent will depend upon the particular compound of formula (III) required: One suitable compound of formula (VI) is that wherein X and $R^d$ are as defined above and $R^c$ represents a formyl group.

An appropriate reagent capable of converting a formyl group to a moiety $R^2$ is an organometallic reagent or Grignard reagent of formula (VII):

$R^3$-Y-M (VII)

wherein $R^3$ and Y are as defined in relation to formula (I), and M represents MgX wherein X is a halogen or M represents a metallo species, especially lithium.

The reaction conditions used in the reaction between the compound of formula (VI) and the appropriate reagent will be those appropriate to the particular nature of the compound of formula (VI) and the reagent. For example the reaction between a compound of formula (VI), wherein $R^c$ represents a formyl group, and an organometallic reagent or Grignard reagent of formula (VII) may be carried out in a solvent such as diethyl ether or tetrahydrofuran at low to ambient temperature.

Suitably $R^3$, in the compound of formula (VII), represents pyridyl.

A compound of formula (VI) may be prepared by reacting a compound of the hereinbefore defined formula (IV) with a compound of formula (VIII):

$R^e$-$CH_2$-$R^x$ (VIII)

wherein $R^e$ is a moiety $R^c$ as defined in relation to formula (VI), or a protected form thereof, and $R^x$ is a leaving group; and thereafter if required removing the protecting group.

Suitably, for preparing compounds of formula (VI) wherein $R^c$ represents a formyl group, $R^e$ is a protected formyl group such as a dialkyl acetal; the protecting group being removed by hydrolysis under mildly acidic conditions.

Suitably, $R^x$ is a halogen atom especially a bromine atom. Thus for example to prepare a compound of formula (VI) wherein $R^c$ represents a formyl group the appropriate compound of formula (IV) may be treated with the known reagent

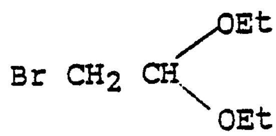

followed by mild acidic hydrolysis.

A compound of formula (I), or a pharmaceutically acceptable salt thereof, or a pharmaceutically acceptable solvate thereof, may also be prepared by reacting a compound of formula (IX):

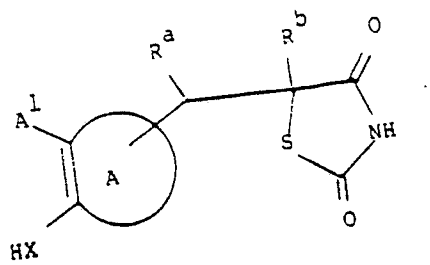

(IX)

wherein A, $R^a$, $R^b$ and X are as defined in relation to formula (I), and $A^1$ is as defined in relation to formula (IV), with a compound of the hereinbefore defined formula (V); and thereafter if required carrying out one or more of the following optional steps:

(i) converting a compound of formula (I) into a further compound of formula (I);

(ii) preparing a pharmaceutically acceptable salt of the compound of formula (I) or a pharmaceutically acceptable solvate thereof.

Preferably $R^2$ in the compound of formula (V) represents $R^{3a}$-Y-Z as defined above.

The reaction between the compounds of formula (v) and (IX) may be carried out under any suitable conditions, for example those described above in relation to the reaction between compounds of formulae (IV) and (V).

A compound of formula (IX) may be prepared by reacting a compound of formula (X):

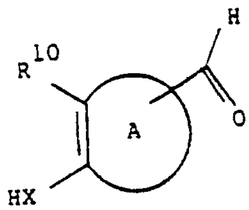

(X)

wherein A and X are as defined in relation to formula (I) and $R^{10}$ represents $A^1$ or a protected form thereof, with 2,4-thiazolidinedione; and thereafter if required removing any protecting group.

Suitable protected forms of the moiety $R^1$-CO- represented by $A^1$ include those wherein the carbonyl group is present in the form of any suitable conventional carbonyl protecting group, such as an acetal or thioacetal group.

Suitable protected forms of any amino group represented by $R^{10}$ include those wherein the amino group is protected by any suitable conventional amino protecting group, such as an acetyl or benzoyl group.

In addition, when $R^{10}$ represents a protected form of an amino group it encompasses those groups which are easily convertible into amino groups, for example a nitro group which is readily convertible into an amino group by reduction, for example catalytic reduction using such catalysts as a palladium carbon catalyst.

The reaction between the compound of formula (X) and 2,4-thiazolidinedione may be carried out under any suitable conditions for example those described above in relation to the reaction between the compounds of formula (III) and 2,4-thiazolidenedione.

A compound of formula (X) wherein $R^{10}$ is a protected form of $A^1$ may be prepared from a compound of the hereinbefore defined formula (IV) and the appropriate reagent required to provide the protected form of the group $A^1$.

It will be appreciated that when $R^{10}$ represents $R^1$-CO or $NH_2$ the compound of formula (X) is a compound of the hereinbefore defined formula (IV).

When $A_1$ in the compound of formula (IX) represents $R^1$-C

and $A^2$ in the compound of formula (v) represents $-CH_2-R^y$, the reaction between the compounds of formulae (IX) and (V) can be envisaged to proceed via an intermediate of formula (IXA):

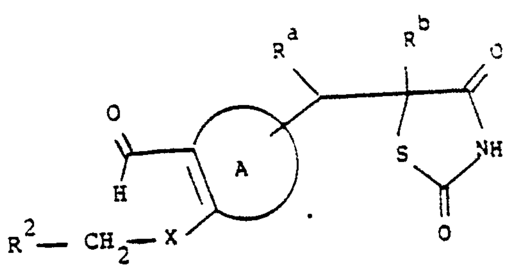

(IXA)

wherein $R^2$, $R^a$, $R^b$, A and X are as defined above.

In certain cases the compounds of formula (IXA) can be isolated and subsequently converted to the appropriate compound of formula (I). We have determined that the isolation of intermediates of the type of (IXA) is facilitated if the $R^2$ moiety is provided in a protected form, specifically those intermediates wherein the moiety Z of $R^2$ is in a protected form.

Accordingly, the present invention further provides a process for preparing a compound of formula (I) wherein $A^0$ represents $R^1$-C -,

or a pharmaceutically acceptable salt thereof, or a pharmaceutically acceptable solvate thereof, which process comprises cyclising a compound of formula (IXB):

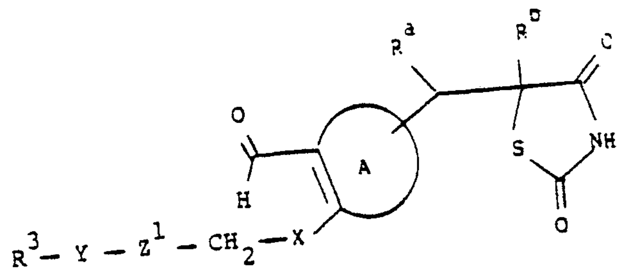

(IXB)

wherein A, $R^3$, $R^a$, $R^b$, X and Y are as defined in relation to formula (I) and $Z^1$ represents Z as defined in relation to formula (I) or a protected form thereof, and thereafter, if required, carrying out one or more of the following optional steps:

(i) removing any protecting group;

(ii) converting a compound of formula (I) into a further compound of formula (I); and

(iii) preparing a pharmaceutically acceptable salt of the compound of formula (I) or a pharmaceutically acceptable solvate thereof.

Preferably, $Z^1$ is a protected form of Z. A preferred example is that wherein $Z^1$ represents a protected carbonyl.

Suitable protected forms of Z represented by $Z^1$ are those comprising conventional protecting groups, for example a carbonyl group is suitably protected as a ketal of type $-C(OR)_2-$ wherein R represents $C_{1-4}$ alkyl, preferably methyl.

The protected forms of Z may be prepared and, when required, the relevant protecting group removed under the appropriate conventional conditions.

The cyclisation of the compound of formula (IXB) may be carried out in any suitable solvent such as an aqueous $C_{1-4}$ alkanol, preferably aqueous ethanol, in the presence of an inorganic acid such as hydrochloric acid at an elevated temperature, conveniently at the reflux temperature of the chosen solvent.

In a further aspect the present invention also provides a process for the preparation of a compound of formula (I), or a pharmaceutically acceptable solvate thereof, wherein $R^a$ and $R^b$ both represent hydrogen, which process comprises reacting a compound of formula (XI):

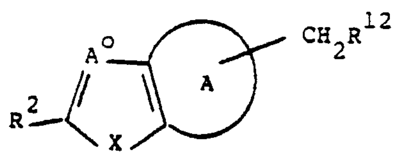

(XI)

wherein $R^2$, $A^0$, A and X are as defined in relation to formula (I) and $R^{12}$ represents a moiety convertible into a 2,4-thiazolidin-5-yl group, with an appropriate reagent capable of converting $R^{12}$ into a 2,4-thiazolidinedione-5-yl group; and thereafter, if required, carrying out one or more of the following optional steps:

(i) converting a compound of formula (I) into a further compound of formula (I);

(ii) preparing a pharmaceutically acceptable salt of a compound of formula (I) or a pharmaceutically acceptable solvate thereof.

Conveniently $R^{12}$ represents a moiety of formula (b):

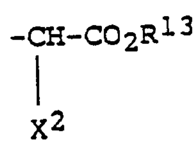

wherein $X^2$ represents a leaving group, such as a halogen atom, and $R^{13}$ represents a $C_{1-6}$ alkyl group. Preferably, $X^2$ represents a chlorine atom.

Preferably $R^{13}$ represents a methyl group.

The nature of the appropriate reagent will of course depend upon the nature of the compound of formula (XI) and especially the nature of $R^{12}$.

One appropriate reagent, especially when $R^{12}$ represents a moiety of the hereinbefore defined formula (b), is thiourea.

The reaction between the compound of formula (XI) and the appropriate reagent will of course depend upon the nature of the two compounds, for example when $R^{12}$ is a moiety of formula (b) and the reagent is thiourea, the reaction is conveniently carried out in a solvent, such as 2-ethoxyethanol, in a temperature range of between 20° C to 135° C, for example 100° C.

The method of preparation chosen to prepare the compound of formula (XI) will depend particularly upon the nature of $R^{12}$, thus for example when $R^{12}$ represents a moiety of formula (b) the compound of formula (XI) may be prepared from a compound of formula (XII):

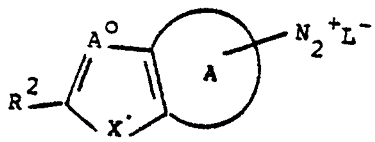

(XII)

wherein $R^2$, $A^0$, A and X are as defined in relation to formula (I) and $L^-$ represents a halide ion, by reaction with a compound of formula (XIII);

$CH_2=CH\text{-}CO_2R^{14}$ (XIII)

wherein $R^{14}$ represents a $C_{1-6}$ alkyl group.

Preferably $L^-$ represents a bromide ion.

The reaction between the compounds of formulae (XII) and (XIII) may be carried out under any suitable conditions, for example in an aqueous solvent, such as aqueous acetone, at a low to ambient temperature, preferably in the range of from -5° C to 50° C, for example 30° C.

Preferably the reaction between the compounds of formulae (XII) and (XIII) is carried out in the presence of a cuprous halide catalyst, for example cuprous iodide.

The compound of formula (XII) may be prepared by diazotization of a compound of formula (XIV):

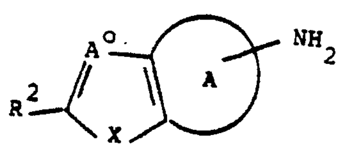

(XIV) .

wherein $R^2$, $A^0$, A and X are as defined in relation to formula (I).

The diazotization of compound (XIV) may be effected under conventional diazotization conditions, for example in a solvent such as aqueous acetone in the presence of a source of nitrite ions, preferably sodium nitrite, at low to ambient temperature, preferably in the range of -20° C to -5° C and preferably in the presence of an acid such as hydrochloric acid.

In a preferred aspect in the preparation of a compound of formula (XI) wherein $R^{12}$ represents a moiety of formula (b), the compound of formula (XII) is not isolated but is reacted in situ with the compound of formula (XIII) to provide a compound of formula (XI).

A compound of formula (XIV) may be prepared by reducing a compound of formula (XV):

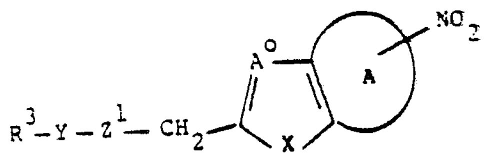

(XV)

wherein $R^3$, $A^0$, A, X and Y are as defined in relation to formula (I) and $Z^1$ is as defined in relation to formula (IXB), and thereafter, if required, removing any protecting group.

The reduction of the compound of formula (XV) may be carried out using any suitable reducing conditions, for example by catalytic reduction using a palladium on carbon catalyst.

A compound of formula (XV) may be prepared by reaction of a compound of formula (XVI):

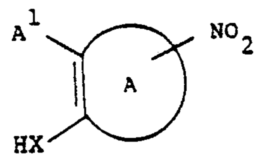

(XVI)

wherein A, $A^1$ and X are as defined in relation to formula (I), with a compound of the hereinbefore defined formula (V), and thereafter, if required, converting Z into a protected form thereof.

The reaction conditions for the reaction between the compounds of formulae (XVI) and (V) are analogous to those used in the preparation of the compound of formula (III) from the compounds of formulae (IV) and (V).

Suitable protected forms of Z include those referred to hereinbefore in respect of $Z^1$ in formula (IXB).

Suitably, when X represents sulphur, the compounds of formula (XVI) may be prepared by reacting a compound of formula (XVIA):

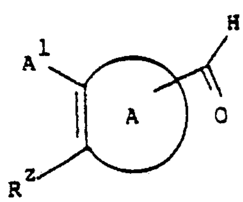

(XVIA)

wherein A and $A^1$ are as defined above and $R^z$ represents a displaceable group or atom, with a source of sulphide ions.

Preferably, $R^z$ represents a halogen atom, for example a chlorine atom.

Preferably, the source of sulphide ions is sodium sulphide.

The reaction between the compound of formula (XVIA) and the source of sulphide ions may be carried out in any suitable aprotic solvent, such as dimethylformamide, at any temperature providing a convenient reaction rate, for example 0 °C.

Conveniently, the compound of formula (XVIA) is not isolated from the reaction mixture, the compound of formula (XVIA) being reacted in situ with the compound of formula (V) to provide a compound of formula (XV).

A compound of formula (XV), wherein $A^0$ represents a moiety $R^1\text{-}\underset{\|}{C}\text{-}$,

may be suitable be prepared by cyclising a compound of formula (XVII):

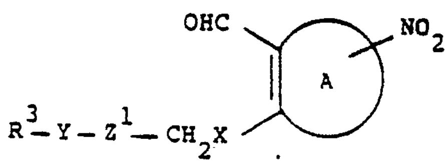

(XVII)

wherein A, $R^3$, X and Y are as defined in relation to formula (I), and Z is as defined in relation to formula (IXB).

The cyclisation of the compound of formula (XVII) may be carried out in any suitable solvent such as an aqueous $C_{1-4}$ alkanol, preferably aqueous ethanol, in the presence of an inorganic acid, such as hydrochloric acid, at an elevated temperature, conveniently at the reflux temperature of the chosen solvent.

The compound of formula (XVII) may be prepared by reacting a compound of formula (XVIII):

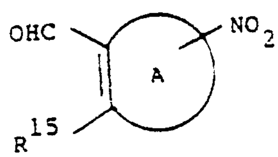

(XVIII)

wherein A is as defined in relation to formula (I) and $R^{15}$ represents either a displaceable group or atom or $R^{15}$ represents a group -XH wherein X is as defined in relation to formula (I), with a compound of formula (XIX):

$R^3$-$Z^1$-$CH_2$-$R^{16}$ (XIX)

wherein $R^3$ is as defined in relation to formula (I), $Z^1$ is as defined in relation to formula (IXB) and $R^{16}$ represents either a group -XH, when $R^{15}$ represents a displaceable group or atom, or $R^{16}$ represents a displaceable group or atom, when $R^{15}$ represents a group -XH.

A suitable displaceable group or atom is a displaceable atom such as a halogen atom, preferably a chlorine atom.

Preferably $R^{15}$ represents a displaceable group or atom and $R^{16}$ represents a group -XH.

The reaction between the compounds of formulae (XVIII) and (XIX) may be carried out using any suitable conditions for example in an aprotic solvent such as dimethylformamide at an ambient or elevated temperature for example at 80° C.

The compounds of formula (XIII) and (XVIA) are either known commercially available compounds or they may be prepared using analogous methods to those used to prepare such compounds.

The compounds of formulae (XVI), (XVIII) and (XIX) are either known compounds or they may be prepared using analogous methods to those used to prepare known compounds for example the methods disclosed in Heterocycles, Vol. 23, No.3, p.633.

The abovementioned conversions of a compound of formula (I) into a further compound of formula (I) include:

(i) the reduction of a compound of formula (I) wherein $R^a$ and $R^b$ together represent a bond to a compound of formula (I) wherein $R^a$ and $R^b$ each represent hydrogen;

(ii) the conversion of a compound of formula (I) wherein Z represents a -CO- group to a compound of formula (I) wherein the said -CO- group is converted to a -CH(OH)- group or a -$CH_2$- group; and

(iii) the conversion of a compound of formula (I) wherein Z represents a -CH(OH)- group to a compound of formula (I) wherein the said -CH(OH)- group is converted to a -$CH_2$- group.

A suitable reduction method for the abovementioned conversion (i) includes catalytic reduction, the use of a complex hydride reducing agent or the use of a metal/solvent reducing system.

Suitable catalysts for use in the catalytic reduction include palladium on carbon catalysts, preferably a 10% palladium on charcoal catalyst; the reduction being carried out in a solvent, for example dioxan, suitably at ambient temperature.

Suitable complex hydride reducing agents include borohydrides, for example sodium borohydride; the reduction being carried out in a solvent such as methanol.

Suitable metal/solvent reducing systems include magnesium and methanol.

Suitably, in the abovementioned conversion (ii), the said -CO- group may be converted into a -CH(OH)- group by reduction, the said compound of formula (I) being treated with an appropriate reducing agent, such as a borohydride reducing agent, especially sodium borohydride, in any suitable solvent, for example methanol, dimethylformamide or mixtures thereof.

Suitably, in the abovementioned conversion (ii), the said -CO- group may be converted into a $-CH_2-$ group by reduction; the said compound of formula (I) being reduced under appropriate reducing conditions such as catalytic reduction, using for example a palladium on carbon catalyst.

Suitably, in the abovementioned conversion (iii) the said -CH(OH)- group may be converted into a methylene group by hydrogenolysis; the said compound of formula (I) being treated under the appropriate hydrogenolysis conditions such as a catalytic hydrogenolysis, using for example a palladium on carbon catalyst.

It will be appreciated from the above that a compound of formula (I), wherein Z represents a -CO- group, may be converted into another compound of formula (I) wherein Z represents a -CH(OH)- group and that such a compound of formula (I) may be converted into a further compound of formula (I) wherein Z represents $-CH_2-$. It will also be appreciated that such conversions may be carried out by reduction of the -CO- group to the -CH(OH)- group followed by hydrogenolysis of the -CH(OH)- group to the $-CH_2-$ group.

The abovementioned conversion of a compound of formula (III) into a further compound of formula (III) encompasses analogous conversions to those described above in relation to the conversion of a compound of formula (I) into a further compound of formula (I). The conversion of a compound of formula (III) into a further compound of formula (III) also includes the conversion of a compound of formula (III) wherein Z represents -CH(OH)- into a compound of formula (III) wherein Z represents -CO-, such conversion being effected via oxidation.

Suitable conditions for the oxidation of a compound of formula (III) wherein Z represents -CH(OH)- include the use of a $CrO_3$-pyridine oxidising agent in a solvent such as dichloromethane.

In any of the abovementioned conversions of the compounds of formula (I) or (III) any reactive groups in the compounds of formula (I) or (III) may be protected by conventional protecting groups. For example the formyl group in the compounds of formula (III) may be protected as an acetal.

Such protecting groups may when necessary be removed under the appropriate conventional conditions, for example the abovementioned acetal group may be removed under mildly acidic conditions.

The present invention also provides a compound of formula (I), or a pharmaceutically acceptable salt thereof, for use as an active therapeutic substance.

The present invention provides a compound of the general formula (I), or a pharmaceutically acceptable salt thereof, or a pharmaceutically acceptable solvate thereof, for use in the treatment of and/or prophylaxis of hyperglycaemia.

In a further aspect the present invention also provides a compound of formula (I), or a pharmaceutically acceptable salt thereof, or a pharmaceutically acceptable solvate thereof, for use in the treatment and/or prophylaxis of hyperlipidaemia.

A compound of the general formula (I), or a pharmaceutically acceptable salt thereof, or a pharmaceutically acceptable solvate thereof, may be administered <u>per se</u> or, preferably, as a pharmaceutical composition also comprising a pharmaceutically acceptable carrier.

Accordingly, the present invention also provides a pharmaceutical composition comprising a compound of the general formula (I), or a pharmaceutically acceptable salt thereof, or a pharmaceutically acceptable solvate thereof, and a pharmaceutically acceptable carrier therefor.

As used herein the term 'pharmaceutically acceptable' embraces compounds, compositions and ingredients for both human and veterinary use: for example the term 'pharmaceutically acceptable salt' embraces a veterinarily acceptable salt.

The composition may, if desired, be in the form of a pack accompanied by written or printed instructions for use.

Usually the pharmaceutical compositions of the present invention will be adapted for oral administration, although compositions for administration by other routes, such as by injection and percutaneous absorption are also envisaged.

Particularly suitable compositions for oral administration are unit dosage forms such as tablets and capsules. Other fixed unit dosage forms, such as powders presented in sachets, may also be used.

In accordance with conventional pharmaceutical practice the carrier may comprise a diluent, filler, disintegrant, wetting agent, lubricant, colourant, flavourant or other conventional adjuvant.

Typical carriers include, for example, microcrystalline cellulose, starch, sodium starch glycollate, polyvinylpyrrolidone, polyvinylpolypyrrolidone, magnesium stearate, sodium lauryl sulphate or sucrose.

Most suitably the composition will be formulated in unit dose form. Such unit dose will normally contain

an amount of the active ingredient in the range of from 0.1 to 1000 mg, more usually 0.1 to 500 mg, and more especially 0.1 to 250 mg.

The present invention further provides a method for the treatment and/or prophylaxis of hyperglycaemia in a human or non-human mammal which comprises administering an effective, non-toxic, amount of a compound of the formula (I), or a pharmaceutically acceptable salt thereof, or a pharmaceutically acceptable solvate thereof, to a hyperglycaemic human or non-human mammal in need thereof.

The present invention further provides a method for the treatment of hyperlipidaemia a human or non-human mammal, which comprises administering an effective, non-toxic, amount of a compound of the general formula (I), or a pharmaceutically acceptable salt thereof, or a pharmaceutically acceptable solvate thereof, to an hyperlipidaemic human or non-human mammal.

Conveniently, the active ingredient may be administered as a pharmaceutical composition hereinbefore defined, and this forms a particular aspect of the present invention.

In the treatment and/or prophylaxis of hyperglycaemic humans, or the treatment and/or prophylaxis of hyperlipidaemic humans, the compound of the general formula (I), or a pharmaceutically acceptable salt thereof, or a pharmaceutically acceptable solvate thereof, may be taken in doses, such as those described above, one to six times a day in a manner such that the total daily dose for a 70 kg adult will generally be in the range of from 0.1 to 6000 mg, and more usually about 1 to 1500 mg.

In the treatment and/or prophylaxis of hyperglycaemic non-human mammals, especially dogs, the active ingredient may be adminstered by mouth, usually once or twice a day and in an amount in the range of from about 0.025 mg/kg to 25 mg/kg, for example 0.1 mg/kg to 20 mg/kg. Similar dosage regimens may be used for the treatment and/or prophylaxis of non-human mammals.

The present invention also provides the use of a compound of formula (I), or a pharmaceutically acceptable salt thereof, or a pharmaceutically acceptable solvate thereof, for the manufacture of a medicament for the treatment and/or prophylaxis of hyperglycaemia.

The present invention further provides the use of a compound of formula (I) or a pharmaceutically acceptable salt thereof, or a pharmaceutically acceptable solvate thereof, for the manufacture of a medicament for the treatment and/or prophylaxis of hyperlipidaemia.

The following Examples illustrate the invention but do not limit it in any way.

Example 1

5-[[2-[(5-Methyl-2-phenyl-4-oxazolyl)hydroxymethyl]benzofuran-5-yl)methyl]-2,4-thiazolidinedione.

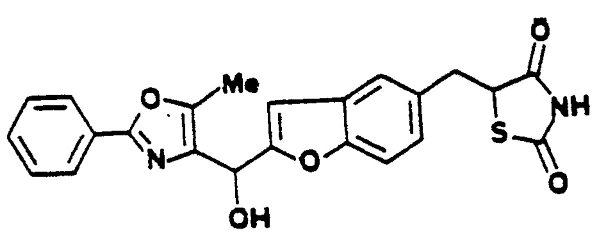

5-[[2-[(5-Methyl-2-phenyl-4-oxazolyl)hydroxymethyl]benzofuran-5-yl]methylene]-2,4-thiazolidinedione (4.49g) in dioxan (450ml) was hydrogenated in the presence of 10% Palladium-charcoal (4.4g) for 5h at room temperature and pressure. A further portion of the catalyst (4.4g) was added, and the hydrogenation continued for a total of 7h. The mixture was filtered through diatomaceous earth and the solvent evaporated to leave a gum which was chromatographed on silica gel with methanol-dichloromethane (1:99) v/v) as eluant to afford the title compound as a foam, m.p. 169-172° C.

$^1H$ n.m.r. δ($CDCl_3$):

2.45(3H,s), 3.20(1H,dd), 3.50(1H,dd), 4.65(1H,dd), 5.85(1H,d), 6.10(1H,d, exchanges with $D_2O$), 6.75(1H,s), 7.13(1H,d), 7.25-7.50(5H,m), 7.90(2H,m) and 11.65(1H,br s, exchanges with $D_2O$).

Example 2

5-[[2-[(5-Methyl-2-phenyl-4-oxazolyl)carbonyl]benzofuran-5-yl]methyl]-2,4-thiazolidinedione.

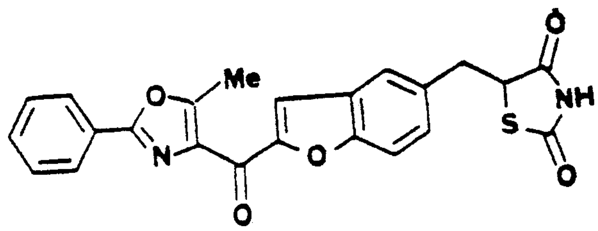

This compound, m.p. 193-6 °C, was prepared from 5-[[2-[(5-methyl-2-phenyl-4-oxazolyl)carbonyl]-benzofuran-5-yl]methylene]-2,4-thiazolidinedione by an analogous procedure to that described in Example 1.

$^1$H n.m.r. $\delta(CDCl_3)$:

2.83(3H,s), 3.30(1H,dd), 3.62(1H,dd), 4.60(1H,dd), 7.30-7.75(6H,m), 8.10(2H,m), 8.57(1H,s) and 8.65(1H, br s, exchanges with $D_2O$)

Example 3

5-[[2-[(5-Methyl-2-phenyl-4-oxazolyl)carbonyl]benzofuran-5-yl]methylene]-2,4-thiazolidinedione.

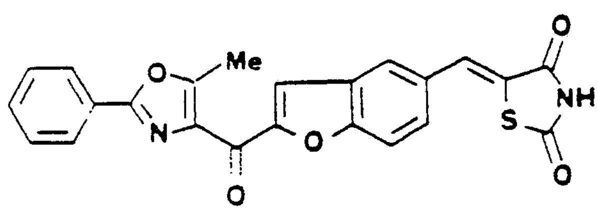

5-Formyl-2-[(5-methyl-2-phenyl-4-oxozolyl)carbonyl] benzofuran (5.84g) and 2,4-thiazolidinedione (2.27g) were dissolved in toluene (250ml) containing piperidine (0.5ml) and benzoic acid (0.5g), and heated to reflux for 4.75h in a Dean and Stark apparatus. On cooling, the title compound crystallised, was collected by filtration and dried in vacuo.

$^1$H n.m.r. $\delta(CDCl_3\text{-}DMSO\text{-}d_6 - D_2O)$:

2.70(3H,s), 7.10-8.30(9H,m) and 8.65(1H,s).

Example 4

5-[[2-[(5-Methyl-2-phenyl-4-oxazolyl)hydroxymethyl]benzofuran-5-yl)methylene]-2,4-thiazolidinedione.

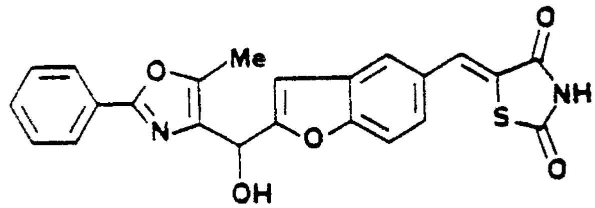

5-[[2-[(5-Methyl-2-phenyl-4-oxazolyl)carbonyl]benzofuran-5-yl]methylene]-2,4-thiazolidinedione (4.30g) was suspended in methanol-dimethylformamide (1:1 v/v, 100ml) at 0°C, and sodium borohydride (0.43g) added with stirring. The mixture was stirred at 0°C for 20 minutes, then poured into ice-cold water (200ml), acidified with glacial acetic acid and the precipitated title compound filtered off, washed with water and dried in vacuo.

$^1$H n.m.r. δ($CDCl_3$-DMSO-$d_6$ 1:1 v/v):

2.40(3H,s), 4.00-6.00(2H,br s, exchanges with $D_2O$), 5.85(1H,s), 6.85(1H,s) and 7.00-8.20(9H,m).

Example 5

5-[[2-(α-Hydroxybenzyl)benzofuran-5-yl]]methyl-2,4-thiazolidinedione

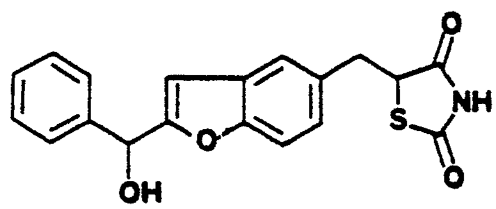

5-[2-(α-Hydroxybenzyl)benzofuran-5-yl]methylene-2,4-thiazolidinedione (2.75g) in dioxan (250ml) was hydrogenated in the presence of 10% palladium-charcoal (2.75g) for 2.75h at room temperature and atmospheric pressure. A further portion of the catalyst (2.75g) was added and the hydrogenation continued overnight. The mixture was filtered through diatomaceous earth and the solvent evaporated to leave a gum. Chromatography on silica gel using methanol-dichloromethane (1.5:98.5 v/v) as eluant afforded the title compound as a foam, m.p. 74-770°C. $^1$H n.m.r. δ ($CDCl_3$):
2.65(1H,s, exchanges with $D_2O$), 3.20(1H,dd), 3.55(1H,dd), 4.55(1H,dd), 5.93(1H,s), 6.50(1H,s), 7.05-7.55-(8H,m), 8.50(1H,s, exchanges with $D_2O$).

Example 6

5-[(2-Benzylbenzofuran-5-yl)methyl]-2,4-thiazolidinedione

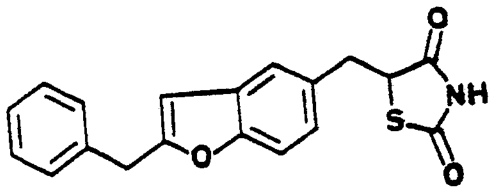

This compound, m.p. 131-132 °C, was isolated from the early fractions during chromatographic purification of the compound of Example 5.

$^1$H n.m.r. δ ($CDCl_3$):

3.15(1H,dd), 3.55(1H,dd), 4.05(2H,s), 4.47(1H,dd), 6.35(1H,s), 7.00-7.50(8H,m), 11.60(1H,broad s, exchanges with $D_2O$).

Example 7

5-[(2-Benzoylbenzofuran-5-yl)methyl]-2,4-thiazolidinedione

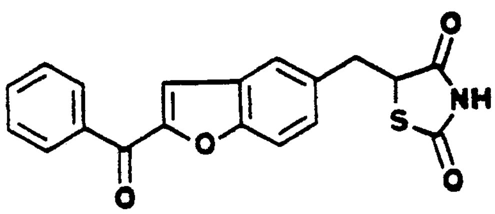

This compound, m.p. 90-92 °C was prepared from 5-[(2-benzoylbenzofuran-5-yl)methylene]-2,4-thiazolidinedione by a procedure analogous to that used in Example 5.

$^1$H n.m.r. δ ($CDCl_3$):

3.55(1H,dd), 3.63(1H,dd), 4.62(1H,dd), 7.30-7.70(7H,m), 8.05(2H,m), 8.25(1H,s, exchanges with $D_2O$).

Example 8

5-[(2-Benzoylbenzofuran-5-yl)methylene]-2,4-thiazolidinedione

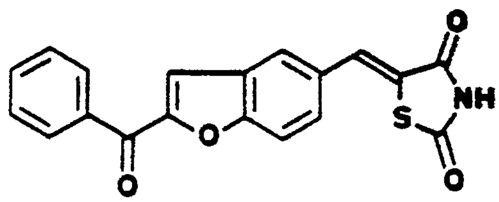

This compound, m.p. 292-40 °C, was prepared from 2-benzoyl-5-formylbenzofuran and 2,4-thiazolidinedione by a procedure analogous to that used in Example 3.

$^1$H n.m.r. δ ($CDCl_3$-DMSO-$d_6$ + $D_2O$):

7.40-8.20 (all H,m).

Example 9

5-[[2-(α-Hydroxybenzyl)benzofuran-5-yl]methylene]-2,4-thiazolidinedione.

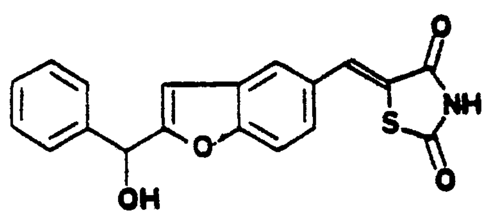

This compound was prepared from 5-[2-benzoylbenzofuran-5-yl)methylene]-2,4-thiazolidinedione by a method analogous to that used in Example 4.

$^1$H n.m.r. δ ($CDCl_3$-DMSO-$d_6$ + $D_2O$):

5.85(1H,s), 6.70(1H,s), 7.10-8.00(9H,m).

Example 10

5-[ 2-[(3-Chlorophenyl)hydroxymethyl]benzofuran-5-yl]methyl]-2,4-thiazolidinedione

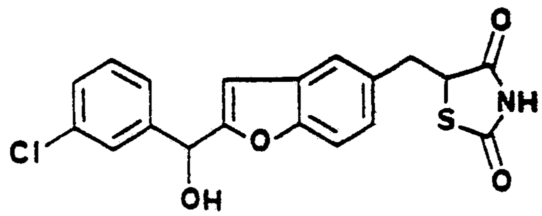

Hydrogenation of 5-[[2-[(3-chlorophenyl)hydroxymethyl] benzofuran-5-yl]methylene]-2,4-thiazolidinedione by the method of Example 5, followed by chromatography on silica gel with methanol-dichloromethane (1.5:98.5 v/v) as eluant afforded the title compound as a foam, m.p. 65° C.

$^{1}$H n.m.r. δ (DMSO-$d_6$):

3.15(1H,dd), 3.40(1H,dd), 4.83(1H,dd), 5.85(1H,d, collapses to singlet on shaking with $D_2O$), 6.35(1H,d, exchanges with $D_2O$), 6.70(1H,s), 7.10-7.55(7H,m), 12.00(1H,broad s, exchanges with $D_2O$).

Example 11

5-[[2-(3-Chlorobenzyl)benzofuran-5-yl]methyl]-2,4-thiazolidinedione

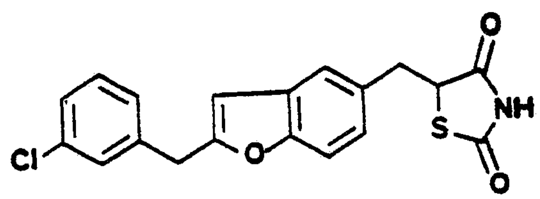

This compound, m.p. 91-4° C, was isolated from the early fractions during chromatographic purification of the compound described in Example 10.

$^{1}$H n.m.r. δ ($CDCl_3$):

3.17(1H,dd), 3.60(1H,dd). 4.07(2H,s), 4.50(1H,dd), 4.80(1H,broad s, exchanges with $D_2O$), 6.40(1H,s), 7.00-7.45(7H,m).

Example 12

5-[[2-(3-Chlorobenzoyl)benzofuran-5-yl]methylene]-2,4-thiazolidinedione

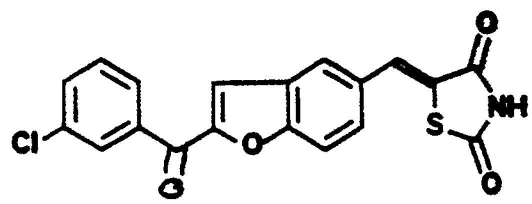

This compound, m.p. 275-6 °C, was prepared from 2-(3-chlorobenzoyl)-5-formylbenzofuran and 2,4-thiazolidinedione by a procedure analogous to that used in Example 3.

$^1$H n.m.r. δ (DMSO-$d_6$):

3.70(1H,broad s, exchanges with $D_2O$), and 7.15-8.10 (9H,m).

Example 13

5-[[2-[(3-Chlorophenyl)hydroxymethyl]benzofuran-5-yl]methylene]-2,4-thiazolidinedione

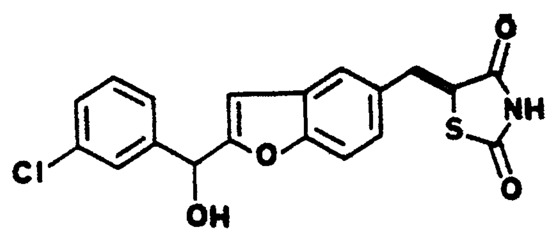

This compound, m.p. 220-222 °C, was prepared from 5-[[2-(3-chlorobenzoyl)benzofuran-5-yl]-methylene]-2,4-thiazolidinedione by a procedure analogous to that used in Example 4.

$^1$H n.m.r. δ ($CDCl_3$-DMSO-$d_6$):

3.50(1H,broad s, exchanges with $D_2O$), 5.82(1H,s), 6.35(1H,broad s, exchanges with $D_2O$), 6.70(1H,s), 7.22-8.00(8H,m).

Example 14

5-[[(2-Benzoyl-3-methylbenzofuran-5-yl)methyl]-2,4-thiazolidinedione

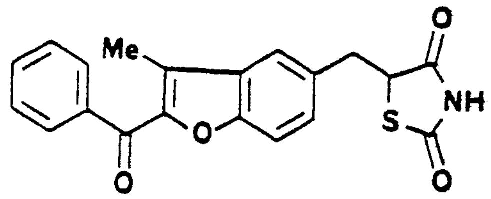

5-[(2-Benzoyl-3-methylbenzofuran-5-yl)methylene]-2,4-thiazolidinedione was hydrogenated according to the method of Example 5. Crystallisation from chloroform-hexane afforded the title compound, m.p. 182-3° C.

$^1$H n.m.r. δ ($CDCl_3$-DMSO-$d_6$):

2.60(3H,s), 3.23(1H,dd), 3.55(1H,dd), 4.57(1H,dd), 7.30-7.70(6H,m), 8.10(2H,m), 11.70(1H,broad s, exchanges with $D_2O$).

Example 15

5-[(2-Benzyl-3-methylbenzofuran-5-yl)methyl]-2,4-thiazolidinedione

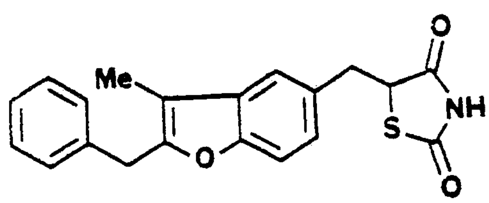

This compound, m.p. 142-5° C was isolated from the reaction mixture when 5-[[2-(α-Hydroxybenzyl)-3-methylbenzofuran-5-yl]methylene]-2,4-thiazolidinedione was hydrogenated by the method of Example 5.

$^1$H n.m.r. δ ($CDCl_3$-DMSO-$d_6$):

2.25(3H,s), 3.17(1H,dd), 3.60(1H,dd), 4.09(2H,s), 4.47(1H,dd), 7.00-7.50(8H,m), 11.50(1H broad s, exchanges with $D_2O$)

Example 16

5-[[2-(α-Hydroxybenzyl)-3-methylbenzofuran-5-yl]methylene]-2,4-thiazolidinedione

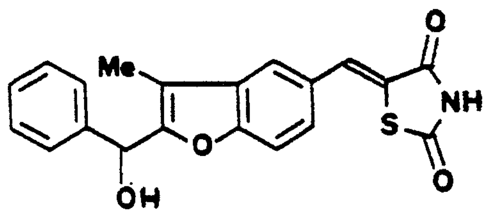

This compound was prepared from 5-[(2-benzoyl-3-methylbenzofuran-5-yl)methylene]-2,4-thiazolidinedione by a procedure analogous to that used in Example 4, and was used in the next stage without further purification.

$^1$H n.m.r. δ ($CDCl_3$-DMSO-$d_6$):

2.40(3H,s), 6.15(1H,s), 6.80(2H,broad s, exchanges with $D_2O$), 7.10-8.10(9H,m).

Example 17

5-[(2-Benzoyl-3-methylbenzofuran-5-yl)methylene]-2,4-thiazolidinedione

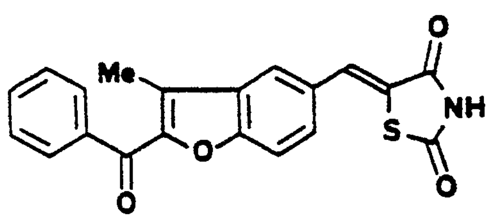

This compound, m.p. 264-5°C was prepared from 2-benzoyl-5-formyl-3-methylbenzofuran and 2,4-thiazolidinedione by a procedure analogous to that used in Example 3.

$^1$H n.m.r. δ ($CDCl_3$-DMSO-$d_6$):

2.55(3H,s), 7.30-8.10(9H,m), 9.50(1H,broad s, exchanges with $D_2O$).

Example 18

5-[[2-[(5-Methyl-2-phenyl-4-oxazolyl)carbonyl]-3-phenylbenzofuran-5-yl]methyl]-2,4-thiazolidinedione.

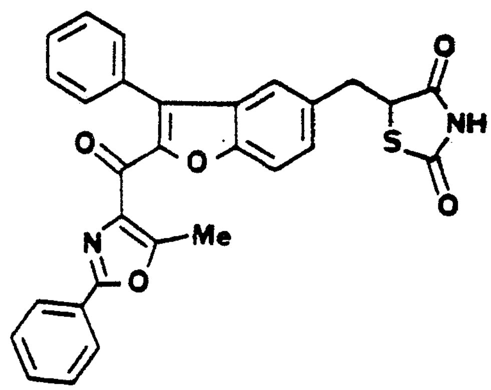

This compound was obtained as a foam, m.p. 125 °C, when 5-[[2-[(5-methyl-2-phenyl-4-oxazolyl)-carbonyl]-3-phenylbenzofuran-5-yl]methylene]-2,4-thiazolidinedione was hydrogenated by a method analogous to that described for example 5.

$^1$H n.m.r. δ ($CDCl_3$):

2.67(3H,s), 3.13(1H,dd), 3.61(1H,dd) 4.45(1H,dd), 7.25-7.90(13H,m), 9.17(1H,broad s, exchanges with $D_2O$).

Example 19

5-[[2-[(5-Methyl-2-phenyl-4-oxazolyl)hydroxymethyl]-3-phenylbenzofuran-5-yl]methyl]-2,4-thiazolidinedione

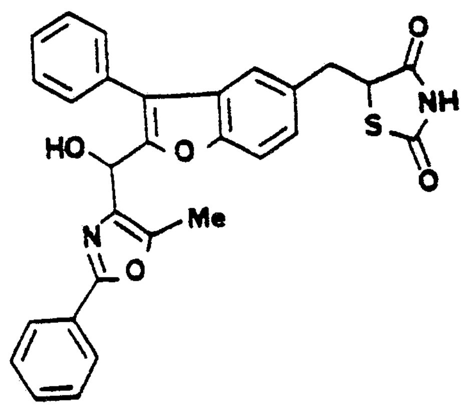

Hydrogenation of 5-[[2-[(5-methyl-2-phenyl-4-oxazolyl)hydroxymethyl]-3-phenylbenzofuran-5-yl]-methylene]-2,4-thiazolidinedione by the method of Example 5 afforded the title compound as a waxy solid which collapsed to a glassy-resin at 110-115 °C.

$^1H$ n.m.r. δ ($CDCl_3$):

2.20(3H,s), 3.15(1H,dd), 3.57(1H,dd), 4.11(1H, exchanges with $D_2O$), 4.49(1H,dd), 6.00(1H,m), 7.10-7.60-(11H,m), 7.95(2H,m), 9.65(1H, broad s, exchanges with $D_2O$).

Example 20

5-[[2-[(5-Methyl-2-phenyl-4-oxazolyl)carbonyl]-3-phenylbenzofuran-5-yl]methylene]-2,4-thiazolidinedione

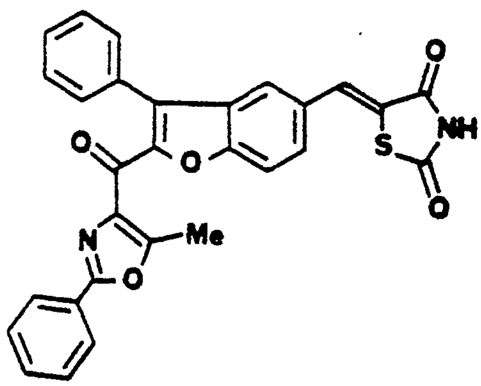

This compound, m.p. 277-8 °C, was prepared from 5-formyl-2-[(5-methyl-2-phenyl-4-oxazolyl)carbonyl]-3-phenylbenzofuran and 2,4-thiazolidinedione by a procedure analogous to that used in Example 3.

$^1H$ n.m.r. δ (DMF-$d_7$-$D_2O$):

2.30(3H,s), 6.90-7.80(14H,m).

Example 21

5-[[2-[(5-Methyl-2-phenyl-4-oxazolyl)hydroxymethyl]-3-phenylbenzofuran-5-yl]methylene]-2,4-thiazolidinedione

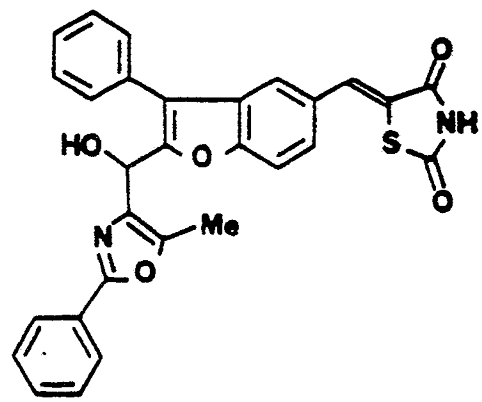

Reduction of 5-[[2-[(5-methyl-2-phenyl-4-oxazolyl)-carbonyl]-3-phenylbenzofuran-5-yl]methylene]-2,4-thiazolidinedione by the method of Example 4 afforded the title compound as a waxy solid which melted to a glassy resin at 110° C.

$^1$H n.m.r. δ (CDCl$_3$):

2.27(3H,s), 5.95(2H, broad s, exchanges with $D_2O$), 6.10(1H,s), 7.25-8.15(14H,m).

Example 22

4-[[7-Methoxy-2-[(5-methyl-2-phenyl-4-oxazolyl) hydroxymethyl]benzofuran-5-yl]methyl]-2,4-thiazolidinedione

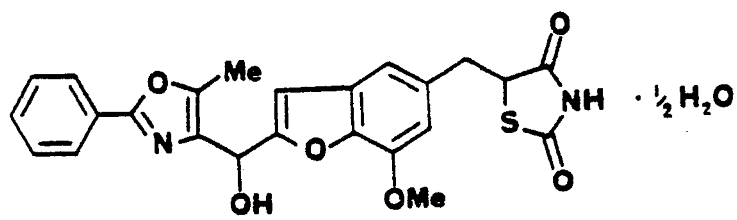

Hydrogenation of 5-[[7-methoxy-2-[(5-methyl-2-phenyl-4-oxazolyl)hydroxymethyl]benzofuran-5-yl]-methylene]-2.4-methylene]-2.4-thiazolidinedione by a method analogous to that used in Example 5 afforded the title compound as a foam. This material melted to a glassy resin at 112-115° C.

$^1$H n.m.r. δ (CDCl$_3$):

2.35(3H,s), 3.15(1H,m), 3.52(1H,m), 3.88(1H,s, exchanges with $D_2O$), 3.93(3H,s), 4.50(1H,dd), 5.90(1H,s), 6.61(1H,m), 6.70(1H,s), 6.95(1H,d), 7.40(3H,m), 7.95(2H,m), 9.40(1H, exchanges with $D_2O$).

Example 23

5-[[7-Methoxy-2-[(5-methyl-2-phenyl-4-oxazolyl)carbonyl]benzofuran-5-yl]methyl]-2-4-thiazolidinedione

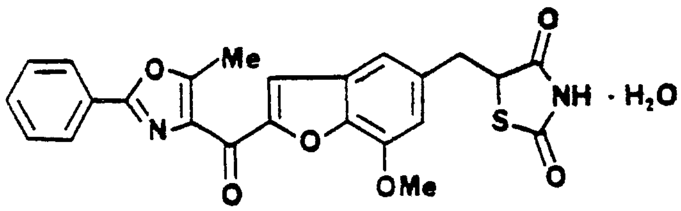

This compound, m.p. 260 °C (decomp.) was prepared from 5-[[7-methoxy-2-[(5-methyl-2-phenyl-4-oxazolyl)carbonyl]benzofuran-5-yl]methylene]-2,4-thiazolidinedione by a procedure analogous to that used in Example 5.

$^1$H n.m.r. δ ($CDCl_3$-DMSO-$d_6$):

2.79(3H,s), 3.17(1H,dd), 3.54(1H,dd), 4.03(3H,s), 4.70(1H,dd), 6.90(1H,s), 7.27(1H,s), 7.50(3H,m), 8.12(2H,m), 8.55(1H,s), 11.80(1H, broad s, exchanges with $D_2O$).

Example 24

5-[[7-Methoxy-2-[(5-methyl-2-phenyl-4-oxazolyl)hydroxymethyl]benzofuran-5-yl]methylene]-2,4-thiazolidinedione

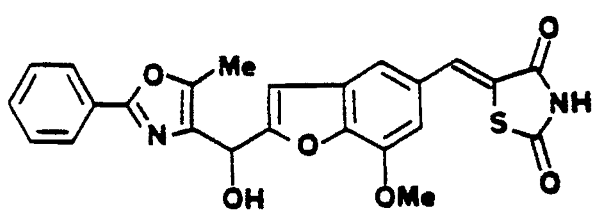

This compound, m.p. 240-242 °C, was prepared from 5-[[7-methoxy-2-[(5-methyl-2-phenyl-4-oxazolyl) carbonyl]benzofuran-5-yl]methylene]-2,4-thiazolidinedione by a method similar to that described in Example 4.

$^1$H n.m.r. δ ($CDCl_3$-DMSO-$d_6$):

2.50(3H,s), 4.00(3H,s), 4.01(2H, broad s, exchanges with $D_2O$), 5.91(1H,s), 6.90-8.20(9H,m).

Example 25

5-[[7-Methoxy-2-[(5-methyl-2-phenyl-4-oxazolyl)carbonyl]benzofuran-5-yl]methylene]-2,4-thiazolidinedione

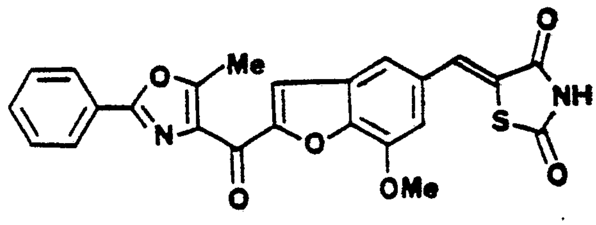

The title compound, m.p. 312-3 °C was prepared from 5-formyl-7-methoxy-2-[(5-methyl-2-phenyl-4-oxazolyl)carbonyl]benzofuran and 2,4-thiazolidinedione by a procedure analogous to that used in Example 3, and was used in the next step without further purification.

Example 26

5-[(2-Benzoylbenzo(b)thien-5-yl)methyl]-2,4-thiazolidinedione

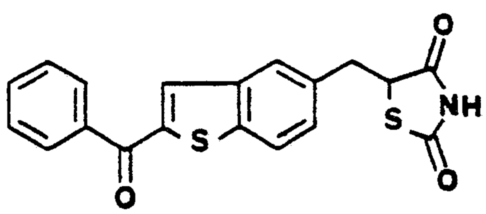

A mixture of methyl 3-(2-benzoylbenzo(b)thien-5-yl)-2-chloropropanoate (7.12g), thiourea (2.28g) and sodium acetate (1.64g) in 2-ethoxyethanol (60ml) was heated at 100 °C for 22h.. Hydrochloric acid (6M; 10ml) was added and heating was continued at 120 °C for 6h. before the mixture was cooled and poured into water (1000ml). The solid was filtered off, washed well with water and dried under vacuum. Chromatography on silica gel with dichloromethane as eluant afforded the title compound as a foam, m.p. 150-153 °C.

$^{1}H$ n.m.r. δ ($CDCl_3$):

3.27(1H,dd), 3.63(1H,dd), 4.62(1H,dd), 7.30-7.95(9H,m), 8.83(1H broad s, exchanges with $D_2O$).

Example 27

5-[[2-[4-Amino-3,5-dichlorobenzoyl]benzofuran-5-yl]methyl]-2,4-thiazolidinedione

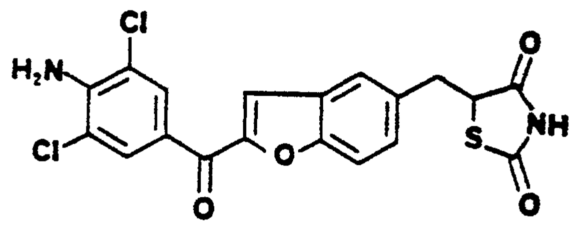

The title compound m.p. 229-31 °C (MeOH-dioxan) was prepared from 5-[[2-[4-amino-3,5-dichlorobenzyl]benzofuran-5-yl]methylene]-2,4-thiazolidinedione by ananalogous procedure to that described in Example 1.

<u>$^1$H n.m.r. δ (DMSO $d_6$ + $D_2O$):</u>

3.25 -3.7(2H, complex), 4.95(1H,dd), 7.4(1H,d), 7.7-7.8(3H,complex), 8.0(2H,s).

Example 28

5-[[2-[4-Amino-3,5-dichlorobenzoyl]benzofuran-5-yl]methylene]-2,4-thiazolidinedione

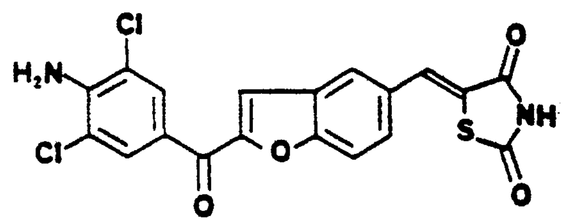

The title compound (m.p. > 320 °C) was prepared from 5-formyl-2-[4-amino-3,5-dichlorobenzoyl]-benzofuran (4.17g) and 2,4-thiazolidinedione (1.64g) by a procedure analogous to that used in Example 3 and was used in the next stage without further purification.

Example 29

5-[[2-[α-Hydroxy-4-amino-3,5-dichlorobenzyl]benzofuran-5-yl]methyl]-2,4-thiazolidinedione

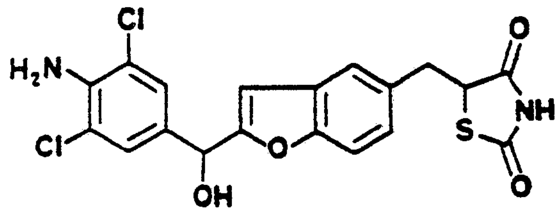

5-[[2-[α-Hydroxy-4-amino-3,5-dichlorobenzyl]benzofuran-5-yl]methylene]-2,4-thiazolidinedione was prepared from 5-[[2-[4-amino-3,5-dichlorobenzoyl]benzofuran-5-yl]methylene]-2,4-thiazolidinedione (3.75g) and sodium borohydride (0.4g) by an analogous procedure to that described in Example 4. This product was hydrogenated as described in Example 5 to give the title compound, m.p. 285 °C.

$^{1}$H n.m.r. δ (DMSO-$d_6$ + $D_2O$):

3.2-3.7(2H,m), 4.85(1H,dd), 5.7(1H,s). 6.45(1H,s), 7.2-7.55(5H,m).

Example 30

5-[[2-(α-Hydroxybenzyl)benzoxazol-5-ylmethyl]-2,4-thiazolidinedione

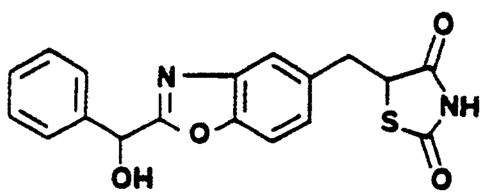

DL- Mandelic acid (1g) and 5-(3-amino-4-hydroxybenzyl)-2,4-thiazolidinedione (1g) were dissolved in xylene (100ml) and heated to reflux for 16h. in a Dean and Stark apparatus. After cooling, the solvent was evaporated and the residue was chromatographed on silica gel with methanol-dichloromethane (5:95 v/v) as eluant to afford the title compound, m.p. 175-178 °C (dichloromethane).

$^{1}$H n.m.r. δ ($CDCl_3$-DMSO-$d_6$ + $D_2O$):

3.45 (1H,dd) 3.55(1H,dd), 4.65(1H,dd) 6.15(1H,s), 7.2-7.9(8H,m).

Example 31

5-[2-(2-Pyridylcarbonyl)benzofuran-5-yl]methyl-2,4-thiazolidinedione.

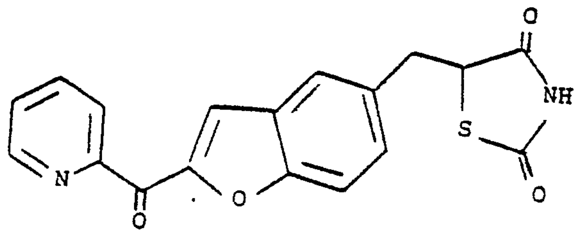

This compound, a foam m.p. 76°C, was isolated as the major product when methyl 3-[2-(2-pyridylhydroxymethyl)benzofuran-5-yl]-2- bromopropanoate was reacted with thiourea by a procedure analogous to that used in Example 26, and the crude reaction mixture was chromatographed on silica gel with methanol-dichloromethane (1:99 v/v) as eluent.

<u>$^1$H n.m.r. δ (CDCl$_3$:</u>

3.33 (1H,dd) 3.63(1H,dd), 4.62(1H,dd), 7.37(1H,dd), 7.50-7.70(3H,m), 7.95(1H,m), 8.25(1H,d), 8.35(1H,br s, exchanges with $D_2O$), 8.47(1H,s), and 8.80(1H,d).

Example XI

5-Formyl-2-[(5-methyl-2-phenyl-4-oxazolyl)carbonyl]benzbenzofuran.

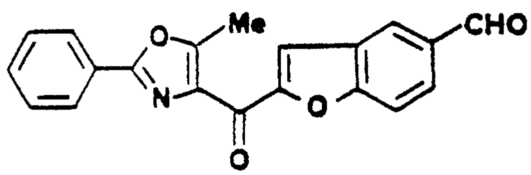

5-Formylsalicylaldehyde (4.50g), potassium carbonate 8.30g) and 4-bromoacetyl-5-methyl-2-phenyloxazole (8.40g) were heated at reflux in 2-butanone (130ml) for 4.5h before being cooled, diluted with ethyl acetate (250ml) washed with water and brine, dried and evaporated to a foam. The foam was chromatographed on silica gel with dichloromethane as eluant to afford the title compound, used directly in the next step.

$^1$H n.m.r. δ (CDCl$_3$):

2.75(3H,s), 7.30-8.40(8H,m), 8.70(1H,s), and 10.15(1H,s).

Example X2

2-Benzoyl-5-formylbenzofuran

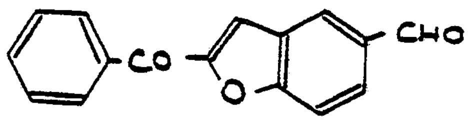

This compound was prepared from 5-formylsalicylaldehyde and α-bromoacetophenone by a method analogous to that used in Example X1.

$^1$H n.m.r. δ ($CDCl_3$):

7.40-8.30(9H,m), 10.15(1H,s).

Example X3

2-(3-Chlorobenzoyl)-5-formylbenzofuran

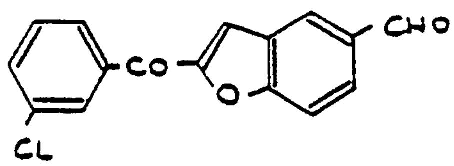

This compound, m.p. 165-6 °C, was prepared from 5-formylsalicylaldehyde and α-bromo-3-chloroacetophenone by a method analogous to that used in Example X1.

$^1$H n.m.r. δ ($CDCl_3$-DMSO-$d_6$):

7.50-8.40(8H,m), 10.15(1H,s).

Example X4

2-Benzoyl-5-cyano-3-methylbenzofuran

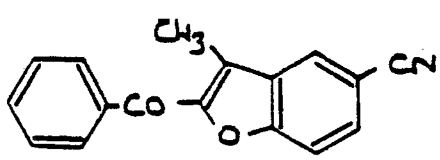

This compound, m.p. 138-9 °C, was prepared from 5-cyano-2-hydroxyacetophenone and α-bromoacetophenone by a method analogous to that used in Example X1.

$^1H$ n.m.r. δ ($CDCl_3$):

2.60(3H,s), 7.50-8.20(8H,m).

Example X5

2-Benzoyl-5-formyl-3-methylbenzofuran

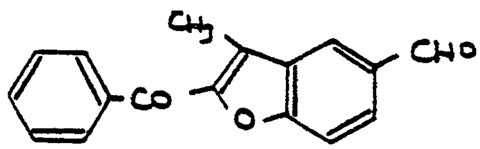

Hydrogen chloride gas was passed into a cooled (cold water bath) suspension of tin (II) chloride (6g) in diethyl ether-ethyl acetate (1:2 v/v; 120ml) for a period of 1.5h.. After this time a slurry of 2-benzoyl-5-cyano-3-methylbenzofuran (5.52g) in diethyl ether-ethyl acetate (1:2 v/v; 90ml) was added, and hydrogen chloride was passed into the resulting stirred suspension for a further 30 minutes. The mixture was stirred for an additional 21h., the solvent was evaporated and the resulting slurry heated at reflux in water (150ml) for 2.5h.. The cooled mixture was extracted with diethyl ether (4x200ml) and the combined ether layers washed with saturated sodium bicarbonate solution and brine, dried over anhydrous magnesium sulphate and evaporated. The resulting solid was chromatographed on silica gel, eluting with hexane-dichloromethane (25:75 v/v) to afford the title compound, m.p. 117°C.

$^1H$ n.m.r. δ ($CDCl_3$):

2.65(3H,s), 7.35-8.30(8H,m), 10.20(1H,s).

Example X6

5-Chloromethyl-2-hydroxybenzophenone.

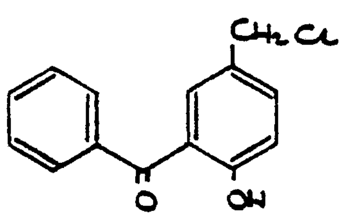

2-Hydroxybenzophenone (50g) was chloromethylated by a procedure analogous to that described in J. Chem. Soc., 1950, 2141 for the chloromethylation of 2-nitrophenol. The title compound and its regioisomer 3-chloromethyl-2-hydroybenzophenone were isolated as a mixture, and were used directly in the next stage.

Example X7

5-Formyl-2-hydroxybenzophenone

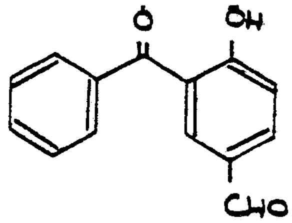

Impure 5-chloromethyl-2-hydroxybenzophenone (48.9g)(from Example X6) was heated at reflux for 2h in 50% aqueous acetic acid (500ml) containing hexamine (50g). Concentrated hydrochloric acid (125ml) was added, and reflux continued for 15 minutes before the mixture was cooled, poured into water (1500 ml) and extracted with dichloromethane (3x500ml). The combined organic layers were washed with brine, dried and evaporated to afford a mixture of the title compound and its regioisomer,3-formyl-2-hydroxybenzophenone, as a mobile oil. This material was used directly in the next step

Example X8

5-Formyl-2-[(5-methyl-2-phenyl-4-oxazolyl)carbonyl)-3-phenylbenzofuran

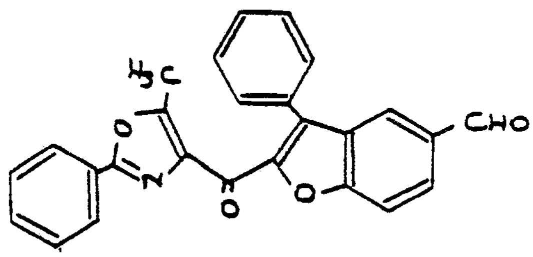

Impure 5-formyl-2-hydroxybenzophenone (10g)(from Example X7) was reacted with 4-bromoacetyl-5-methyl-2-phenyloxazole (12.4g) by a method analogous to that described in Example X1. The title compound was obtained after chromatography as a mixture containing the isomeric 7-benzoyl-2-[(5-methyl-2-phenyl-4-oxazolyl)carbonyl]benzofuran. This solid was used in the next step without further purification.

Example X9

5-Formyl-7-methoxy-2-[(5-methyl-2-phenyl-4-oxazolyl)carbonyl]benzofuran

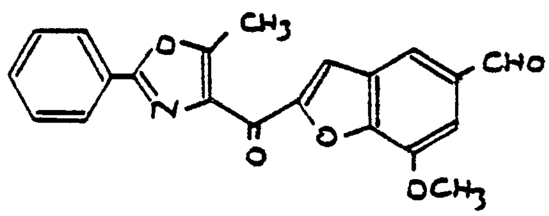

This compound, m.p. 211-213° C, was prepared from 5-formyl-3-methoxysalicylaldehyde and 4-bromoacetyl-5-methyl-2-phenyloxazole by a method analogous to that used in Example X1.

$^1$H n.m.r. δ ($CDCl_3$-DMSO-$d_6$):

2.81(3H,s), 4.05(3H,s), 7.30-8,15(7H,m), 8.70(1H,s), 10.10(1H,s).

Example X10

2-Benzoyl-5-nitrobenzothiophene

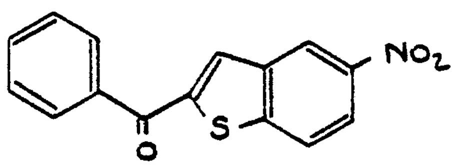

A solution of sodium sulphide (21.12g) in water (50ml) was added dropwise over 30 minutes to a mechanically stirred ice-cooled solution of 2-chloro-5-nitrobenzaldehyde (14.9g) in dimethylformamide (160 ml). The mixture was stirred for a further 15 minutes at 0° C before the addition of a solution of α-chloroacetophenone (12.96g) in dimethylformamide (50 ml). The resulting mixture was stirred at room temperature for 30 minutes before being poured into water(1500 ml). The precipitate was filtered off, washed with water and dried under vacuum to afford a solid, m.p. 203-4° C.

$^1$H n.m.r. δ ($CDCl_3$ - $CF_3CO_2D$):

7.30-8.80(all H,m).

Example X11

5-Amino-2-benzoylbenzothiophene

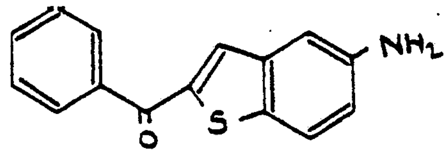

A suspension of 2-benzoyl-5-nitrobenzothiophene (10g) in methanol (500ml) was hydrogenated over 10% palladium-charcoal (10g) for 7h.. The mixture was filtered through diatomaceous earth and the solvent evaporated to afford the title compound as a gummy solid which was used directly in the next step.

<u>$^1$H n.m.r. $\delta$ ($CDCl_3$:-DMSO-$d_6$):</u>

4.70(2H, broad s, exchanges with $D_2O$), 6.80-7.90(9H,m).

Example X12

Methyl 3-(2-benzoylbenzo(b)thien-5-yl)-2-chloropropanoate

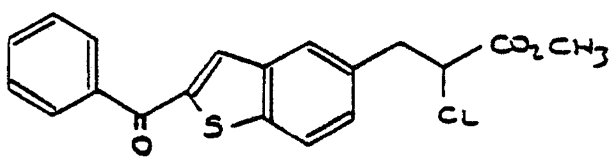

A solution of sodium nitrite (2g) in water (5ml) was slowly added to an ice-cooled mixture of 5-amino-2-benzoylbenzothiophene (5.75g), acetone (60ml) and concentrated hydrochloric acid (6ml) at such a rate that the reaction temperature was maintained below 5°C. The resulting thick precipitate was diluted with water (2ml) and acetone (5ml) and stirred manually at 0°C for 30 minutes. Methyl acrylate (14ml) was added and the mixture warmed to 30°C with portionwise addition of copper (I) iodide (0.2g), and then stirred at room temperature for 2h. before evaporation of the solvent. The residue was dissolved in dichloromethane (200ml), washed with water (3x200ml) and brine, dried over anhydrous Magnesium Sulphate and evaporated to give an oil. This was chromatographed on silica gel with hexane-dichloromethane (15:85 v/v) as eluant to afford the title compound as an oil which was used in the next step without further purification.

$^1$H n.m.r. $\delta$ ($CDCl_3$):

3.35(2H,dd), 3.67(3H,s), 4.45(1H,t), 7.15-8.00(9H,m).

Example X13

5-Formyl-2-[(4-amino-3,5-dichlorobenzoyl]benzofuran

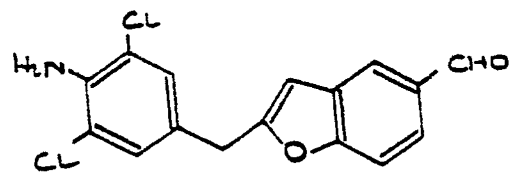

The title compound (m.p. 226° C) was prepared from 5-formylsalicylaldehyde (3.0g) and 4-amino-3, 5-dichloro-α-bromoacetophenone (5.66g) by the method described in Example X1, and was used in the next stage without purification.

Example X14

2-[2,2-Dimethoxy-2-(2-pyridyl)ethoxy]-5-nitrobenzaldehyde

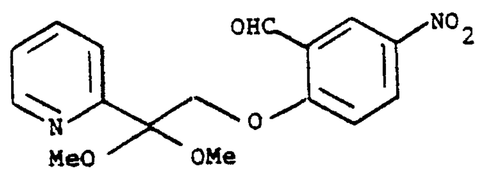

Sodium hydride (60% dispersion in mineral oil, 4g) was added in portions to a stirred solution of 2,2-dimethoxy-2-(2-pyridyl)ethanol (15g) in dimethylformamide (200ml) under nitrogen. Stirring was continued for 1 hour prior to the addition of 2-chloro-5- nitrobenzaldehyde (15.3g) in dimethylformamide (200ml) over 30 minutes. The mixture was heated at 80° C for 21 hours, then cooled, quenched by the addition of methanol (5ml) and the solvent evaporated. The residue was suspended in ethyl acetate (1 litre), washed with water (4X1 litre), brine (1 litre), dried ($MgSO_4$) and evaporated to afford a gum which was chromatographed on silica gel with methanol-dichloromethane (1.5:98.5 v/v) to afford the title compound, m.p. 132-5° C

$^1$H n.m.r. δ ($CDCl_3$):

3.35(6H,s), 4.71(2H,s), 7.00-8.70 (7H,m) and 10.15(1H,s).

Example X15

5-Nitro-2-(2-pyridylcarbonyl)benzofuran

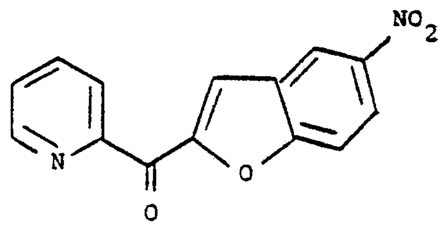

2-(2,2-Dimethoxy-2-(2-pyridyl)ethoxy]-5-nitrobenzaldehyde (11.5g) was heated at reflux for 17 hours in a mixture of ethanol (250ml) concentrated hydrochloric acid (125ml) and water (125ml). The solvent was evaporated and the residue then suspended in saturated aqueous sodium bicarbonate (500ml) and extracted with dichloromethane (3x300ml). The combined organic layers were washed with brine, dried ($MgSO_4$) and evaporated to afford the title compound, m.p. 210-212°C, which was used directly in the next step.

$^1H$ n.m.r. $\delta$ ($CDCl_3$):

7.50-8.85 (8H,m)
IR (KBr disc): 1645cm$^{-1}$ (ketone).

Example X16

5-Amino-2-(2-pyridylhydroxymethyl)benzofuran

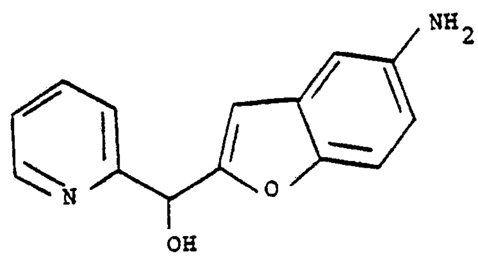

5-Nitro-2-(2-pyridylcarbonyl)benzofuran (8.54g) was suspended in methanol (400ml) and hydogenated in the presence of 10% palladium-charcoal (10g) for 20 hours. The mixture was filtered through diatomaceous earth and the solvent evaporated to afford the title compound as a gum. This material was used in the next stage without further purification.

$^1H$ n.m.r. $\delta$ ($CDCl_3$ - DMSO-$d_6$):

4.20(3H,br s), exchanges with $D_2O$), 5.82(1H,s), 6.20-7.60(7H,m) and 8.52(1H,d).

Example X17

Methyl 3-[2-(2-pyridylhydroxymethyl)benzofuran-5-yl]-2-bromopropanoate

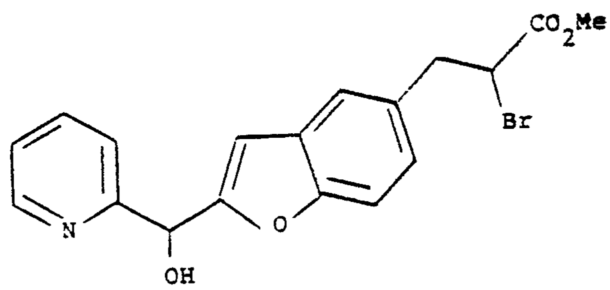

Sodium nitrite (1.73g) in water (4ml) was added dropwise to a stirred, ice-cooled mixture of 5-amino-2-(2-pyridylhydroxymethyl)benzofuran (4.68g) and 47% hydrobromic acid (10.2ml) in methanol (20ml) and acetone (50ml) at a rate such that the reaction temperature did not exceed 5° C. The mixture was stirred at this temperature for 20 minutes prior to the addition of methyl acrylate (11.8ml). The mixture was warmed to 38° C and copper (I) iodide (0.26g) added portionwise with stirring. After a further 2 hours the mixture was evaporated and the residue suspended in 880 ammonia (150ml) and extracted with ethyl acetate (3x150ml). The combined organic layers were washed with water and brine, dried and evaporated to afford a gum. This material was chromatographed on silica gel with methanol-dichloromethane (1:99 v/v) as eluent to afford the title compound as a viscous oil.

$^1$H n.m.r. δ ($CDCl_3$):

3.00-3.75(2H,m), 3.66(3H,s), 4.37(1H,t), 4.90(1H,br s, exchanges with $D_2O$), 5.92(1H,s), 6.61(1H,s), 7.00-7.80(6H,m) and 8.55(1H,d).

DEMONSTRATION OF EFFICACY OF COMPOUNDS

Obese Mice, Oral Glucose Tolerance Test.

C57b1/6 obese (ob/ob) mice were fed on powdered oxoid diet. After at least one week, the mice continued on a powdered oxoid diet or were fed powdered oxoid diet containing the test compound. After 8 days on the supplemented diet all of the mice were fasted for 5 hours prior to receiving an oral load of glucose (3 g/kg). Blood samples for glucose analysis were taken 0, 45, 90 and 135 minutes after glucose administration and the results appear below as the percentage reduction in area under the blood glucose curve where test compound treated groups are compared with the control groups. 7 mice were used for each treatment.

| EXAMPLE NO: | LEVEL IN DIET (mmol $kg^{-1}$ of DIET) | %REDUCTION IN AREA UNDER BLOOD GLUCOSE CURVE |
|---|---|---|
| 1 | 0.3 | 62 |
| 2 | 2.0 | 63 |
| 3 | 0.3 | 12 |
| 5 | 1.0 | 43 |
| 6 | 1.0 | 51 |
| 7 | 0.3 | 46 |
| 10 | 0.3 | 29 |
| 11 | 0.3 | 29 |
| 14 | 0.3 | 19 |
| 15 | 0.3 | 42 |
| 18 | 0.3 | 11 |
| 19 | 0.3 | 34 |
| 22 | 0.3 | 33 |
| 23 | 0.3 | 27 |
| 26 | 0.3 | 19 |
| 27 | 1.0 | 50 |
| 29 | 0.3 | 47 |
| 30 | 0.3 | 49 |

Toxicity

No toxicological effects were indicated for any of the compounds of the invention in any of the abovementioned tests.

**Claims**

1. A compound of formula (I):

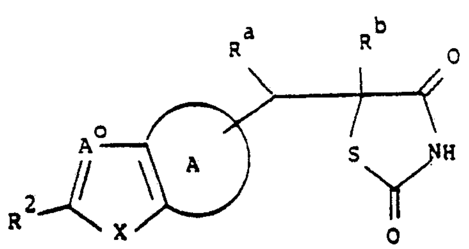

(I)

or a pharmaceutically acceptable salt thereof, or a pharmaceutically acceptable solvate thereof, character-

ized in that:
$A^0$ represents nitrogen or a moiety $R^1$- C -

wherein
$R^1$ represents hydrogen, alkyl or a substituted or unsubstituted aryl group;
$R^2$ represents a moiety $R^3$-Y-Z- wherein $R^3$ represents substituted or unsubstituted phenyl, substituted or unsubstituted pyridyl or a substituted or unsubstituted oxazolyl group, and Y represents $-(CH_2)_n$- wherein n represents zero or any integer in the range of 1 to 6 and Z represents $-CH_2$-,-CH(OH)- or -CO-;
$R^a$ and $R^b$ each represent hydrogen or $R^a$ and $R^b$ together represent a bond;
A represents a residue of a benzene ring, the carbon atoms of the residue having in total up to four substituents;
and X represents O or S.

2. A compound according to claim 1, wherein $A^0$ represents a moiety $R^1$-C-.

3. A compound according to claim 2 or claim 3, wherein $R^1$ represents hydrogen.

4. A compound according to any one of claims 1 to 3, wherein $R^2$ represents $R^3$-$CH_2$-, $R^3$-CH(OH)- or $R^3$-CO-.

5. A compound according to any one of claims 1 to 4, wherein $R^3$ represents phenyl.

6. A compound according to any one of claims 1 to 4, wherein $R^3$ represents a moiety of formula (a):

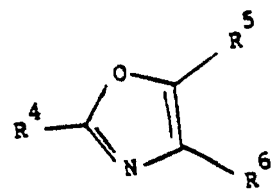

(a)

wherein $R^4$ represents an alkyl group or substituted or unsubstituted aryl and either $R^5$ represents a bond and $R^6$ represents an alkyl group or $R^5$ represents an alkyl group and $R^6$ represents a bond.

7. A compound according to any one of claims 1 to 6, wherein $R^a$ and $R^b$ each represent hydrogen.

8. A compound according to any one of claims 1 to 7, wherein X represents O.

9. A compound of formula (II):

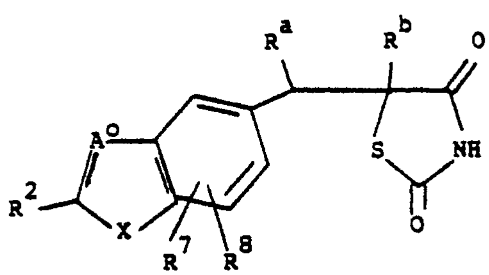

(II)

or a pharmaceutically acceptable salt thereof, or a pharmaceutically acceptable solvate thereof, wherein $A^0$, $R^2$, $R^a$, $R^b$ and X are as defined in relation to formula (I) in claim 1, and $R^7$ and $R^8$ each independently represent hydrogen, alkyl, alkoxy or halogen.

10. A compound according to claim 9, wherein $R^8$ represents hydrogen or methoxy.

11. A compound according to claim 1, selected from the list consisting of:
5-[[2-[(5-methyl-2-phenyl-4-oxazolyl)hydroxymethyl]benzofuran-5-yl)methyl]-2,4-thiazolidinedione;
5-[[2-[(5-methyl-2-phenyl-4-oxazolyl)carbonyl]benzofuran-5-yl]methyl]-2,4-thiazolidinedione;
5-[[2-[(5-methyl-2-phenyl-4-oxazolyl)carbonyl]benzofuran-5-yl]methylene]-2,4-thiazolidinedione;
5-[[2-[(5-methyl-2-phenyl-4-oxazolyl)hydroxymethyl]benzofuran-5-yl)methylene]-2,4-thiazolidinedione;
5-[[2-(α-hydroxybenzyl)benzofuran-5-yl]]methyl-2,4-thiazolidinedione;
5-[(2-benzylbenzofuran-5-yl)methyl]-2,4-thiazolidinedione;
5-[(2-benzoylbenzofuran-5-yl)methyl]-2,4-thiazolidinedione;

5-[(2-benzoylbenzofuran-5-yl)methylene]-2,4-thiazolidinedione;
5-[[2-(α-hydroxybenzyl)benzofuran-5-yl]methylene]-2,4-thiazolidinedione;
5-[2-[(3-chlorophenyl)hydroxymethyl]benzofuran-5-yl]methyl]-2,4-thiazolidinedione;
5-[[2-(3-chlorobenzyl)benzofuran-5-yl]methyl]-2,4-thiazolidinedione;
5-[[2-(3-chlorobenzoyl)benzofuran-5-yl]methylene]-2,4-thiazolidinedione;
5-[[2-[(3-chlorophenyl)hydroxymethyl]benzofuran-5-yl]methylene]-2,4-thiazolidinedione;
5-[[(2-benzoyl-3-methylbenzofuran-5-yl)methyl]-2,4-thiazolidinedione;
5-[(2-benzyl-3-methylbenzofuran-5-yl)methyl]-2,4-thiazolidinedione;
5-[[2-(α-hydroxybenzyl)-3-methylbenzofuran-5-yl]methylene]-2,4-thiazolidinedione;
5-[(2-benzoyl-3-methylbenzofuran-5-yl)methylene]-2,4-thiazolidinedione;
5-[[2-[(5-methyl-2-phenyl-4-oxazolyl)carbonyl]-3-phenylbenzofuran-5-yl]methyl]-2,4-thiazolidinedione;
5-[[2-[(5-methyl-2-phenyl-4-oxazolyl)hydroxymethyl]-3-phenylbenzofuran-5-yl]methyl]-2,4-thiazolidinedione;
5-[[2-[(5-methyl-2-phenyl-4-oxazolyl)carbonyl]-3-phenylbenzofuran-5-yl]methylene]-2,4-thiazolidinedione;
5-[[2-[(5-methyl-2-phenyl-4-oxazolyl)hydroxymethyl]-3-phenylbenzofuran-5-yl]methylene]-2,4-thiazolidinedione;
4-[[7-methoxy-2-[(5-methyl-2-phenyl-4-oxazolyl)hydroxymethyl]benzofuran-5-yl]methyl]-2,4-thiazolidinedione;
5-[[7-methoxy-2-[(5-methyl-2-phenyl-4-oxazolyl)carbonyl]benzofuran-5-yl]methyl]-2-4-thiazolidinedione;
5-[[7-methoxy-2-[(5-methyl-2-phenyl-4-oxazolyl)hydroxymethyl]benzofuran-5-yl]methylene]-2,4-thiazolidinedione;
5-[[7-methoxy-2-[(5-methyl-2-phenyl-4-oxazolyl)-carbonyl]benzofuran-5-yl]methylene]-2,4-thiazolidinedione;
5-[(2-benzoylbenzo(b)thien-5-yl)methyl]-2,4-thiazolidinedione;
5-[[2-[4-amino-3,5-dichlorobenzoyl]benzofuran-5-yl]methyl]-2,4-thiazolidinedione;
5-[[2-[4-amino-3,5-dichlorobenzoyl]benzofuran-5-yl]methylene]-2,4-thiazolidinedione;
5-[[2-[α-hydroxy-4-amino-3,5-dichlorobenzyl]benzofuran-5-yl]methyl]-2,4-thiazolidinedione;
5-[[2-(α-hydroxybenzyl)benzoxazol-5-yl]methyl]-2,4-thiazolidinedione; and
5-[2-(2-pyridylcarbonyl)benzofuran-5-yl]methyl-2,4-thiazolidinedione; or a pharmaceutically acceptable salt thereof or a pharmaceutically acceptable solvate thereof.

12. A process for the preparation of a compound of formula (I), or a pharmaceutically acceptable salt thereof, or a pharmaceutically acceptable solvate thereof, which process comprises either:

(a) reacting a compound of formula (III):

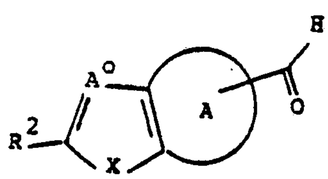

(III)

wherein $A^0$, A, $R^2$ and X are as defined in relation to formula (I), with 2,4-thiazolidinedione; or

(b) reacting a compound of formula (IX):

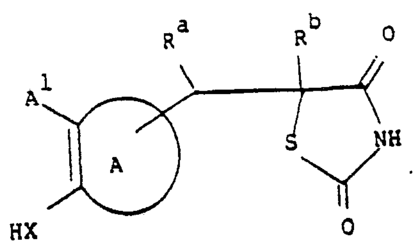

(IX)

wherein $A^0$, A, $R^a$ and $R^b$ are as defined in relation to formula (I), with a compound of the hereinbefore defined formula (V); or

(c) for preparing a compound of formula (I) wherein $A^0$ represents $R^1$ -C-,

or a pharmaceutically acceptable salt thereof, cyclising a compound of formula (IXB):

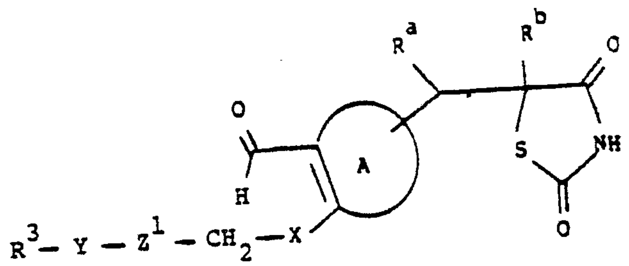

(IXB)

wherein A, $R^3$, $R^a$, $R^b$, X and Y are as defined in relation to formula (I), $A^1$ is as defined in relation to formula (IV) and $Z^1$ represents Z as defined in relation to formula (I) or a protected form thereof, or
(d) reacting a compound of formula (XI):

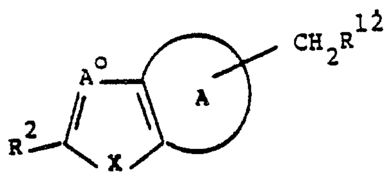

(XI)

wherein $R^2$, $A^0$, A and X are as defined in relation to formula (I) and $R^{12}$ represents a moiety convertible into a 2,4-thiazolidin-5-yl group, with an appropriate reagent capable of converting $R^{12}$ into a 2,4-thiazolidinedione-5-yl group;
and thereafter if required carrying out one or more of the following optional steps:

(i) removing any protecting group;

(ii) converting a compound of formula (I) into a further compound of formula (I);

(iii) preparing a pharmaceutically acceptable salt of the compound of formula (I) or a pharmaceutically acceptable solvate thereof.

13. A pharmaceutical composition comprising a compound of formula (I), or a pharmaceuticlly acceptable salt thereof, or a pharmaceutically acceptable solvate thereof, and a pharmaceutically acceptable carrier therefor.

14. A compound of formula (I), or a pharmaceutically acceptable salt thereof, or a pharmaceutically açceptable solvate thereof for use as an active therapeutic substance.

15. A compound of formula (I), or a pharmaceutically acceptable salt thereof, or a pharmaceutically acceptable solvate thereof, for use in the treatment of and/or prophylaxis of hyperglycaemia or hyperlipidaemia.

16. The use of a compound of formula (I), or a pharmaceutically acceptable salt thereof, or a pharmaceutically acceptable solvate thereof, for the manufacture of a medicament for the treatment and/or prophylaxis of hyperglycaemia or hyperlipidaemia.

Claims for the following Contracting State: ES:

1. A process for preparing a compound of formula (I):

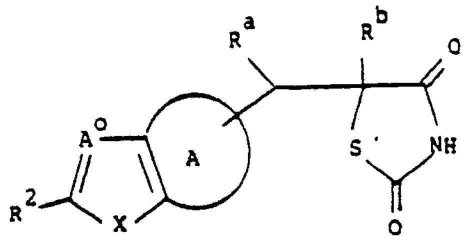

(I)

or a pharmaceutically acceptable salt thereof, or a pharmaceutically acceptable solvate thereof, wherein:
$A^0$ represents nitrogen or a moiety $R^1$- C -

wherein
$R^1$ represents hydrogen, alkyl or a substituted or unsubstituted aryl group;
$R^2$ represents a moiety $R^3$-Y-Z- wherein $R^3$ represents substituted or unsubstituted phenyl, substituted or unsubstituted pyridyl or a substituted or unsubstituted oxazolyl group, and Y represents $-(CH_2)_n-$ wherein n represents zero or any integer in the range of 1 to 6 and Z represents $-CH_2-$,-CH(OH)- or -CO-;
$R^a$ and $R^b$ each represent hydrogen or $R^a$ and $R^b$ together represent a bond;
A represents a residue of a benzene ring, the carbon atoms of the residue having in total up to four substituents;
and X represents O or S;
which process comprises either:
(a) reacting a compound of formula (III):

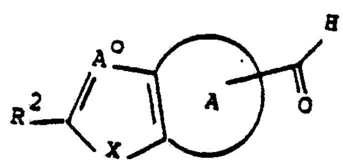

(III)

wherein $A^0$, A, $R^2$ and X are as defined in relation to formula (I), with 2,4-thiazolidinedione; or
(b) reacting a compound of formula (IX):

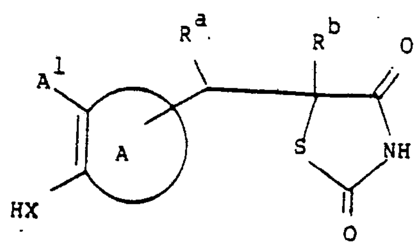

(IX)

wherein $A^0$, A, $R^a$ and $R^b$ are as defined in relation to formula (I), with a compound of the hereinbefore defined formula (V); or
(c) for preparing a compound of formula (I) wherein $A^0$ represents $R^1$ -C-,

or a pharmaceutically acceptable salt thereof, cyclising a compound of formula (IXB):

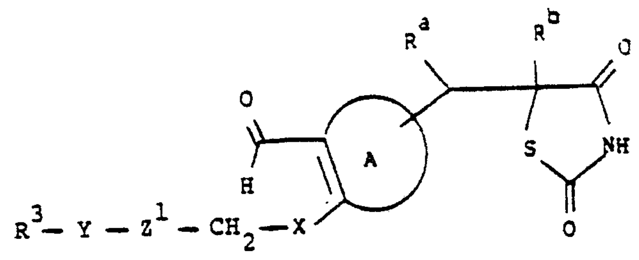

(IXB)

wherein A, $R^3$, $R^a$, $R^b$, X and Y are as defined in relation to formula (I), $A^1$ is as defined in relationto formula (IV) and $Z^1$ represents Z as defined in relation to formula (I) or a protected form thereof, or

(d) reacting a compound of formula (XI):

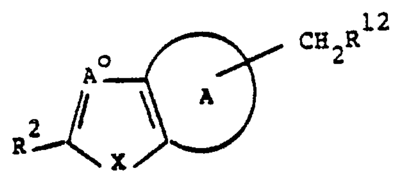

(XI)

wherein $R^2$, $A^0$, A and X are as defined in relation to formula (I) and $R^{12}$ represents a moiety convertible into a 2,4-thiazolidin-5-yl group, with an appropriate reagent capable of converting $R^{12}$ into a 2,4-thiazolidinedione-5-yl group;

and thereafter if required carrying out one or more of the following optional steps:

(i) removing any protecting group;

(ii) converting a compound of formula (I) into a further compound of formula (I);

(iii) preparing a pharmaceutically acceptable salt of the compound of formula (I) or a pharmaceutically acceptable solvate thereof.

2. A process according to claim 1, wherein $A^0$ represents a moiety $R^1$-C-.

3. A process according to claim 2 or claim 3, for preparing a compound wherein $R^1$ represents hydrogen.

4. A process according to any one of claims 1 to 3, for preparing a compound wherein $R^2$ represents $R^3$-$CH_2$-, $R^3$-CH(OH)- or $R^3$-CO-.

5. A process according to any one of claims 1 to 4, for preparing a compound wherein $R^3$ represents phenyl.

6. A process according to any one of claims 1 to 4, for preparing a compound wherein $R^3$ represents a moiety of formula (a):

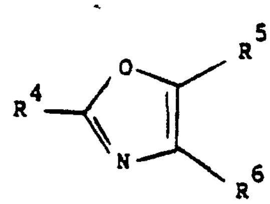

(a)

wherein $R^4$ represents an alkyl group or substituted or unsubstituted aryl and either $R^5$ represents a bond and $R^6$ represents an alkyl group or $R^5$ represents an alkyl group and $R^6$ represents a bond.

7. A process according to any one of claims 1 to 6, for preparing a compound wherein $R^a$ and $R^b$ each represent hydrogen.

8. A process according to any one of claims 1 to 7, for preparing a compound wherein X represents O.

9. A process for preparing a compound of formula (II):

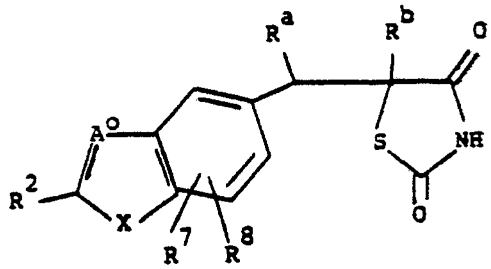

(II)

or a pharmaceutically acceptable salt thereof, wherein $A^0$, $R^2$, $R^a$, $R^b$ and X are as defined in relation to formula (I) in claim 1, and $R^7$ and $R^8$ each independently represent hydrogen, alkyl, alkoxy or halogen.

10. A process according to claim 9, for preparing a compound wherein $R^8$ represents hydrogen or methoxy.

11. A process according to claim 1, for preparing a compound selected from the list consisting of:
5-[[2-[(5-methyl-2-phenyl-4-oxazolyl)hydroxymethyl]benzofuran-5-yl)methyl]-2,4-thiazolidinedione;
5-[[2-[(5-methyl-2-phenyl-4-oxazolyl)carbonyl]benzofuran-5-yl]methyl]-2,4-thiazolidinedione;
5-[[2-[(5-methyl-2-phenyl-4-oxazolyl)carbonyl]benzofuran-5-yl]methylene]-2,4-thiazolidinedione;
5-[[2-[(5-methyl-2-phenyl-4-oxazolyl)hydroxymethyl]benzofuran-5-yl)methylene]-2,4-thiazolidinedione;
5-[[2-(α-hydroxybenzyl)benzofuran-5-yl]]methyl-2,4-thiazolidinedione;
5-[(2-benzylbenzofuran-5-yl)methyl]-2,4-thiazolidinedione;
5-[(2-benzoylbenzofuran-5-yl)methyl]-2,4-thiazolidinedione;
5-[(2-benzoylbenzofuran-5-yl)methylene]-2,4-thiazolidinedione;
5-[[2-(α-hydroxybenzyl)benzofuran-5-yl]methylene]-2,4-thiazolidinedione;
5-[2-[(3-chlorophenyl)hydroxymethyl]benzofuran-5-yl]methyl]-2,4-thiazolidinedione;
5-[[2-(3-chlorobenzyl)benzofuran-5-yl]methyl]-2,4-thiazolidinedione;
5-[[2-(3-chlorobenzoyl)benzofuran-5-yl]methylene]-2,4-thiazolidinedione;
5-[[2-[(3-chlorophenyl)hydroxymethyl]benzofuran-5-yl]methylene-2,4-thiazolidinedione;
5-[[(2-benzoyl-3-methylbenzofuran-5-yl)methyl]-2,4-thiazolidinedione;
5-[(2-benzyl-3-methylbenzofuran-5-yl)methyl]-2,4-thiazolidinedione;
5-[[2-(α-hydroxybenzyl)-3-methylbenzofuran-5-yl]methylene-2,4-thiazolidinedione;
5-[(2-benzoyl-3-methylbenzofuran-5-yl)methylene]-2,4-thiazolidinedione;
5-[[2-[(5-methyl-2-phenyl-4-oxazolyl)carbonyl]-3-phenylbenzofuran-5-yl]methyl]-2,4-thiazolidinedione;
5-[[2-[(5-methyl-2-phenyl-4-oxazolyl)hydroxymethyl]-3-phenylbenzofuran-5-yl]methyl]-2,4-thiazolidinedione;
5-[[2-[(5-methyl-2-phenyl-4-oxazolyl)carbonyl]-3-phenylbenzofuran-5-yl]methylene]-2,4-thiazolidinedione;
5-[[2-[(5-methyl-2-phenyl-4-oxazolyl)hydroxymethyl]-3-phenylbenzofuran-5-yl]methylene]-2,4-thiazolidinedione;
4-[[7-methoxy-2-[(5-methyl-2-phenyl-4-oxazolyl)hydroxymethyl]benzofuran-5-yl]methyl]-2,4-thiazolidinedione;
5-[[7-methoxy-2-[(5-methyl-2-phenyl-4-oxazolyl)carbonyl]benzofuran-5-yl]methyl]-2-4-thiazolidinedione;
5-[[7-methoxy-2-[(5-methyl-2-phenyl-4-oxazolyl)hydroxymethyl]benzofuran-5-yl]methylene]-2,4-thiazolidinedione;
5-[[7-methoxy-2-[(5-methyl-2-phenyl-4-oxazolyl)carbonyl]benzofuran-5-yl]methylene]-2,4-thiazolidinedione;
5-[(2-benzoylbenzo(b)thien-5-yl)methyl]-2,4-thiazolidinedione;
5-[[2-[4-amino-3,5-dichlorobenzoyl]benzofuran-5-yl]methyl]-2,4-thiazolidinedione;
5-[[2-[4-amino-3,5-dichlorobenzoyl]benzofuran-5-yl]methylene]-2,4-thiazolidinedione;
5-[[2-[α-hydroxy-4-amino-3,5-dichlorobenzyl]benzofuran-5-yl]methyl]-2,4-thiazolidinedione;
5-[[2-(α-hydroxybenzyl)benzoxazol-5-yl]methyl]-2,4-thiazolidinedione; and
5-[2-(2-pyridylcarbonyl)benzofuran-5-yl]methyl-2,4-thiazolidinedione; or a pharmaceutically acceptable salt thereof or a pharmaceutically acceptable solvate thereof.

12. A compound of formula (I), or a pharmaceutically acceptable salt thereof, or a pharmaceutically acceptable solvate thereof, for use as an active therapeutic substance.

13. A compound of formula (I), or a pharmaceutically acceptable salt thereof, or a pharmaceutically acceptable solvate thereof, for use in the treatment of and/or prophylaxis of hyperglycaemia or hyperlipidaemia.

14. The use of a compound of formula (I), or a pharmaceutically acceptable salt there of or a pharmaceutically acceptable solvate thereof, for the manufacture of a medicament for the treatment and/or prophylaxis of hyperglycaemia or hyperlipidaemia.

European Patent Office

# EUROPEAN SEARCH REPORT

Application Number

EP 88 30 5285

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | EP-A-0 207 605 (PFIZER INC.)<br>* Claims 1-5,10 * | 1-5,7-10,13-16 | C 07 D 417/06<br>C 07 D 417/14<br>A 61 K 31/425 |
| D,A | EP-A-0 193 256 (TAKEDA)<br>* Claims * | 1,13-16 | |
| D,A | EP-A-0 177 353 (TAKEDA)<br>* Claims * | 1,13-16 | |
| | | | TECHNICAL FIELDS SEARCHED (Int. Cl.4) |
| | | | C 07 D 417/00 |

The present search report has been drawn up for all claims

| Place of search | Date of completion of the search | Examiner |
|---|---|---|
| THE HAGUE | 16-09-1988 | HENRY J.C. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)